(19) Europäisches Patentamt
European Patent Office
Office européen des brevets

(11) EP 2 809 327 B1

(12) # EUROPEAN PATENT SPECIFICATION

(45) Date of publication and mention of the grant of the patent:
**01.08.2018 Bulletin 2018/31**

(21) Application number: **13701646.5**

(22) Date of filing: **30.01.2013**

(51) Int Cl.:
***A61K 31/718*** (2006.01) ***A61P 35/00*** (2006.01)
***A61K 31/7068*** (2006.01) ***A61K 31/4045*** (2006.01)
***A61K 45/06*** (2006.01) ***A61K 31/337*** (2006.01)

(86) International application number:
**PCT/EP2013/051778**

(87) International publication number:
**WO 2013/113747 (08.08.2013 Gazette 2013/32)**

(54) **HYDROXYALKYL STARCH FOR THE TREATMENT OF CANCERS BY REDUCTION OF TUMOR GROWTH RATES**

HYDROXYLALKYLSTÄRKE FÜR DIE BEHANDLUNG VON KREBS

HYDROXYALKYLE DE L'AMIDON POUR LE TRAITEMENT DU CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.01.2012 EP 12153068**
**30.01.2012 US 201261592017 P**

(43) Date of publication of application:
**10.12.2014 Bulletin 2014/50**

(73) Proprietor: **Fresenius Kabi Deutschland GmbH**
**61352 Bad Homburg (DE)**

(72) Inventors:
- **WESTPHAL, Martin**
**61350 Bad Homburg (DE)**
- **BAASNER, Silke**
**Schöneck 61137 (DE)**

(74) Representative: **Fresenius Kabi Deutschland GmbH**
**Patent Department**
**Borkenberg 14**
**61440 Oberursel (DE)**

(56) References cited:
**DE-A1- 4 023 788 US-B1- 6 207 654**

- **LIANG HUA ET AL: "The effects of preloading infusion with hydroxyethyl starch 200/0.5 or 130/0.4 solution on hypercoagulability and excessive platelet activation of patients with colon cancer", BLOOD COAGULATION & FIBRINOLYSIS, vol. 21, no. 5, July 2010 (2010-07), pages 406-413, XP009160709,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 809 327 B1

## Description

## Background of the invention

Cancer, tumor-associated diseases and neoplastic disease states are serious and often life-threatening conditions. These diseases, which are characterized uncontrolled proliferation, also called cell proliferating diseases, are also a focal point of many research projects, devoted to identifying new active therapeutic ingredients which prove to be effective in the treatment of these diseases. Such active ingredients prolong the life expectancy of the patient, inhibit the rapidly progressing cell growth associated with the neoplasm, or bring about regression of the neoplasm, or improve quality of life.

Hydroxyalkyl starches (HAS) are polymers which are derived from natural base materials and are modified. HAS are prepared from amylopectin-rich starches. The parent starch may be branched or unbranched, or may consist of a mixture of both. Hydroxyethyl starches (HES) are based almost exclusively on amylopectin, in other words on branched chains of glucose molecules.

The medical use of hydroxyalkyl starches, and more particularly of hydroxyethyl starch, is known. It is used in particular in volume therapy as a plasma substitute, and also in clinical haemodialysis (Sommermeyer et al., 1987, Krankenhauspharmazie, 8(8): 271-278; Weidler et al., 1991, Arzneimittelforschung/Drug Research, 41: 494-498). Intravenous administration of a hydroxyethyl starch solution, which allows the erythrocytes, (red blood corpuscles), to continue transporting oxygen through the body, can be used, for example, in order to prevent a state of shock following severe blood loss caused by trauma or by surgery.

DE4023788 (Schumann) describes the use of hydroxyethyl starch for the treatment of damage of the inner ear. Specifically it discloses the use of hydroxyethyl starch in a treatment called hyperbaric oxygen therapy. Such a treatment is applied to treat tinnitus and acute hearing loss and it is suggested to use it in treating cancer patients. The only example in this application describes how a HES solution is applied to patient in an oxygen pressure chamber, but fails to demonstrate or discuss any therapeutic effect. In this context, the patent application also suggests the use of hydroxylethyl starch for the treatment of head and neck tumors, but without showing any evidence or a theoretical connection between hearing loss after acoustic incidence and head and neck cancer.

Additionally it has been proposed to use hydroxyethyl starch (HES) to introduce active pharmaceutical ingredients into the peritoneum. In some treatment regimens of peritoneal carcinomatosis the cytotoxic or cytostatic drugs are applied locally. Here it has been shown that the local use of solutions containing HES results in higher retention times of the cytotoxic or cytostatic drug in the peritoneum, compared to the use of dialysis solutions free of osmotically active colloids (Mohamed et al (2003) European Journal of Surgical Oncology vol 29, p 261-265).

It has also been suggested to use HES as an absorbable barrier, as an anti-adhesion agent in injured body cavities (WO 96/40108).

Many of the usual cytotoxic or cytostatic ingredients, together referred to as cytostatica, these being active ingredients which inhibit cell growth, that are used in present-day cancer therapy have only low water solubility. This presents problems for their administration. The low water-solubility must typically be overcome by means of complex formulation techniques, such as by the addition of various excipients, which in general entail toxic side-effects. One possible solution proposed has been the coupling of cytostatica to macromolecular carriers, such as hydroxyethylated starch, for example, in order to enable the administration of so called polymeric prodrugs.

Besides the enhancement of the water solubility of the drug, prodrugs have been proposed to provide an advantageous targeting and/or an enhancement of the stability of the therapeutic agent. Further, such prodrugs were suggested to prolong the circulation lifetime, to provide an extended duration of activity, or to achieve a reduction of side effects and drug toxicity. Thus, besides the preparation of prodrugs of water insoluble cytotoxic or cytostatic agents, providing prodrugs of water soluble cytotoxic or cytostatic agents is also of high interest in order to modify the onset and/or duration of action of the cytotoxic agent *in vivo.*

WO 03/074088 describes hydroxyethyl starch conjugates with, for example, cytostatica such as daunorubicin, wherein the cytostatica are usually directly coupled via an amine group to the hydroxyethyl starch yielding in 1:1 conjugates. However no use of these conjugates *in vivo* was shown therein.

US 6,207,654 (Zikria) teaches that hydroxyethyl starch may be used to improve a cancer treatment based on administration of interleukins, by reducing the side effects of interleukin 2 (IL-2). It is explained that the polysaccharide molecules act as endothelial membrane stabilizers. Due to their biophysical/biochemical properties, they seem to prevent leakage of serum protein from capillary endothelial junctions by sealing these and thereby stabilizing the capillary membranes.

Currently, the routine cancer treatment relies on three treatment options, surgery, radiation therapy and drug therapy, also referred to as chemotherapy. Chemotherapy involves the administration of drugs, which are designed to kill highly proliferating cells, such as cancer cells, or tumor cells, or at least to stop them from proliferating any further. They are commonly referred to as cytostatica. These drugs however are not selective in killing only the cancer cells.

Hence this type of treatment is associated with severe side effects for the patient. A non-comprehensive list of side effects comprises anemia, nausea, vomiting, appetite changes, diarrhea, constipation, fatigue, pain, hair loss, bleeding, swelling, increased susceptibility for infection, reduced memory, nerve changes, mouth and throat changes, sexual and fertility changes, skin and nail, urination problems. These side effects can be so severe, that the treatment with cytostatics has to be stopped due to the high toxicity, in order to keep the patient alive. However, during these phases of recovery, wherein the patient may regain some general health, the tumor often also recovers and starts to grow again. The problem of the high toxicity of cytostatic drugs used to treat cancer patients is well known. Hence there is a need in the art for a treatment option for cancer patients, which inhibits progression of cancer while not stressing or impairing the subject in need of treatment any further.

An ideal cancer treatment would target the tumor growth and the tumor cell proliferation selectively. Healthy cell proliferation at a normal and controlled rate would be unaffected.

It is one aspect of the current invention to provide for a treatment that is effective in the treatment of cancer and at the same time shows no or significantly less toxic side effects for the treated patient than when given cytostatics. This task has been solved by the provision of hydroxyethylated starch(es) for the treatment of cancer by reducing tumor growth rates. It could be shown that these substances have a growth rate limiting effect on tumors, but do not harm normal cells. While HES solutions have been administered to a high number of humans (without tumors) without showing any severe side effect, it was now for the first time noticed that these substances might have an antiproliferative effect selectively on tumor cells.

It has been shown that the sole administration of hydroxyethylated starch to a subject suffering from cancer in form of a growing tumor, inhibited its further progression by reducing the size of the tumor, associated with said cancer, compared to the size of tumors in untreated subjects, measured at the end of the treatment/non-treatment phase. This significant therapeutic effect of administered hydroxyethyl starches in reducing tumor growth rate, whilst not causing a decreased general health status, indicated by a loss of body weight, has been demonstrated herein for the first time. To our knowledge, no one has ever reported upon a therapeutic effect of the hydroxyethylated starch itself on a cancer or a tumor. Whilst hydroxyethylated starches have been proposed as stabilizing agents or solubilisers or osmotically active ingredients, it has never been shown that the application of HES itself has a tumor growth rate reducing effect.

According to the invention hydroxyethylated starch(es) are provided as therapeutically active compounds for treatment of cancer, characterized by the presence of growing tumors, which are therapeutically effective in reducing the tumor growth rate, preferably whilst not affecting a normally proliferating cell. In another aspect of the invention a pharmaceutical composition comprising a hydroxyethylated starch as therapeutically active ingredient, resulting in a reduction of tumor growth rates, is provided for the treatment of cancer.

According to the invention, the term "cancer" refers to a proliferative disorder or disease caused or characterized by the proliferation of cells which have lost susceptibility to normal growth control. The term encompasses a disease which is associated with the growing of tumors and any other cell proliferative disorders. According to the invention, the term is meant to include all pathological conditions involving uncontrolled growth of cells, irrespective of stage or of invasiveness.

In one embodiment the cancer may be localized to a specific tissue or organ (e.g. in the breast, the prostate or the lung), and, thus, may not have spread beyond the tissue of origin. In another embodiment the cancer may be invasive, and, thus may have spread beyond the layer of tissue in which it originated into the normal surrounding tissues (frequently also referred to as locally advanced cancer). Invasive cancers may or may not be metastatic. In a preferred embodiment the cancer is metastatic. A cancer is metastatic, if it has spread from its original location to distant parts of the body.

Further the term cancer is understood to describe all types of cancer known in the art.

The term "tumor" is meant to describe an accumulation of cells that are growing in an uncontrolled manner, an abnormally grown or growing body tissue, or an accumulation of proliferating cells. Tumors can be cancerous (malignant) or noncancerous (benign). A cell proliferative disease usually results in the occurrence of a tumor.

A "pharmaceutical composition" according to the invention comprises a therapeutically effective amount of a HES, as described herein, which can be further substituted, for example via the hydroxyl function attached at the alkyl rest, or instead of this hydroxyl function, and preferably of all those HES that are specifically and explicitly disclosed, including thio-HES.

The pharmaceutical composition may comprise solid or liquid formulations of different concentrations. Different embodiments comprising the hydroxyl ethylated starch either on its own or as a pharmaceutical composition are described in more detail below: According to the invention the active ingredient, hydroxyethyl starch may be administered on its own, simply dissolved in an electrolytic solution, or it may be used in combination with a pharmaceutical excipient. Generally, the hydroxyethyl starch itself will be in a solid form which can be combined with a suitable pharmaceutical excipient that can be in either solid or liquid form. As excipients, carbohydrates, inorganic salts, antimicrobial agents, antioxidants, surfactants, buffers, acids, bases, and combinations thereof may be mentioned. A carbohydrate such as a sugar, a derivatized sugar such as an alditol, aldonic acid, an esterified sugar, and/or a sugar polymer may be present

as an excipient. Specific carbohydrate excipients include, for example: monosaccharides, such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol, sorbitol (glucitol), pyranosyl sorbitol, myoinositol, and the like. The excipient may also include an inorganic salt or buffer such as citric acid, sodium chloride, potassium chloride, sodium sulfate, potassium nitrate, sodium phosphate monobasic, sodium phosphate dibasic, and combinations thereof.

The pharmaceutical composition according to the present invention may also comprise an antimicrobial agent for preventing or determining microbial growth, such as, e.g., benzalkonium chloride, benzethonium chloride, benzyl alcohol, cetylpyridinium chloride, chlorobutanol, phenol, phenylethyl alcohol, phenylmercuric nitrate, thimersol, and combinations thereof.

The pharmaceutical composition according to the present invention may also comprise an antioxidant, such as, e.g., ascorbyl palmitate, butylated hydroxyanisole, butylated hydroxytoluene, hypophosphorous acid, monothioglycerol, propyl gallate, sodium bisulfite, sodium formaldehyde sulfoxylate, sodium metabisulfite, and combinations thereof.

The pharmaceutical composition according to the present invention may also comprise a surfactant, such as, e.g., polysorbates, or pluronics sorbitan esters; lipids, such as phospholipids and lecithin and other phosphatidylcholines, phosphatidylethanolamines, acids and fatty esters; steroids, such as cholesterol; and chelating agents, such as EDTA or zinc.

The pharmaceutical composition according to the present invention may also comprise acids or bases such as, e.g., hydrochloric acid, acetic acid, phosphoric acid, citric acid, malic acid, lactic acid, formic acid, trichloroacetic acid, nitric acid, perchloric acid, phosphoric acid, sulfuric acid, fumaric acid, and combinations thereof, and/or sodium hydroxide, sodium acetate, ammonium hydroxide, potassium hydroxide, ammonium acetate, potassium acetate, sodium phosphate, potassium phosphate, sodium citrate, sodium formate, sodium sulfate, potassium sulfate, potassium fumarate, and combinations thereof. Generally, the excipient will be present in a pharmaceutical composition according to the present invention in an amount of 0.001 to 99.999 wt.-%, preferably from 0.01 to 99.99 wt.-%, more preferably from 0.1 to 99.9 wt.-%, in each case based on the total weight of the pharmaceutical composition.

In a preferred embodiment pharmaceutical compositions according to the invention comprise the hydroxyethyl starch, as described herein, as the only pharmaceutically active ingredient.

In another preferred embodiment the pharmaceutical composition may comprise further therapeutically effective ingredients, wherein HES is therapeutically active against cancer, by reducing the tumor growth rate.

The terms "treating cancer" and "treatment of cancer", refer to therapeutic measures, wherein the object is to prevent or to slow down (lessen) an undesired physiological change or disorder, such as the growth, development or spread of a hyperproliferative condition, such as a cell proliferative disease or a neoplastic disease, the forming of a benign or malignant tumor, or metastases therefrom, or cancer. For purposes of this invention, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission whether partial or total. Metastatic cancer cells usually arise from a cell type disclosed herein and the major difference from the types disclosed herein is that these cells are now present in a tissue from which the cancer cells did not originally develop. Consequently, if a cancer type is mentioned the term encompasses its metastatic form.

It is to be understood that a treatment can also mean prolonging survival as compared to expected survival if not receiving treatment. It is to be understood that a treatment can also be understood as prevention of cancer or prevention of tumor growth.

In a preferred embodiment the treatment is effective to reduce the growth rate of tumors arising from metastatic cancers.

It is particularly envisaged that the term "treatment of cancer" according to the invention comprises the administration of a therapeutically effective amount of the aforementioned compounds resulting in at least one of the effects from the group consisting of reducing the number of cancer cells; reducing the tumor size; inhibiting i.e., slowing to some extent and preferably stopping cancer cell infiltration into peripheral organs; inhibiting i.e., slowing to some extent and preferably stopping tumor metastasis; inhibiting, at least to some extent, tumor growth; and relieving to some extent one or more of the symptoms associated with cancer. Whether a particular amount of the aforementioned compounds exerts at least one or several of these effects i.e. is pharmaceutically effective, can be determined by well known measures. Particularly, it can be determined by assessing cancer therapy efficacy. Cancer therapy efficacy, e.g., can be assessed by determining the time to disease progression, the increasing of quality of life, and/or by determining the response rate. Thus, the required dosage will depend on the severity of the condition being treated, the patient’s individual response, the method of administration used, the cancer type, the tumor and the like. The skilled person is able to establish a correct dosage based on his general knowledge. Generally, the dose may also be administered independently from the state of the disease as the product is considered as non-toxic and dose limits are considered to be based on the current clinical experience (with e.g. Voluven® 10% solution of HES 130/0.4: 30ml/kg/day and/or Volulyte® 6% solution of HES 130/0.4: 50ml/kg/day).

The term "administering" as used herein, preferably, refers to the introduction of the hydroxyethyl starch according to the invention, or the pharmaceutical composition according to the invention, into subjects, such as cancer patients. The term comprises methods for administering a particular compound by parenteral and enteral routes of administration. The parenteral routes of administration are selected from the group comprising intravascular, transmucosal, trans-/intradermal, intramuscular (i.m.), intravenous (i.v.), intradermal, subcutaneous (s.c.), intraperitoneal (i.p.), intraventricular, intracranial, vaginal, nasal, intratumoral, intraosseal, intrathecal and intrapulmonal. The enteral methods of administration are selected from the group comprising oral, nasal, sublingual and rectal, administration and administration by gavage (via feeding tube), such as a percutaneous endoscopic gastrostomy (PEG tube) or percutaneous jejunostomy feeding tube (PJG tube). It is to be understood that the route of administration may depend on the cancer to be treated.

According to the invention the preferred route of administration is the parenteral administration. It is further preferred that such parenteral route is an infusion, preferably into a blood vessel. The most preferred route of administration is an intravenous route. Preferably, the administration of a single dose (bolus) of a therapeutically effective amount of the aforementioned compounds is over a period of 5 min to 5 h.

Hydroxyethylated starches for the treatment and prophylaxis of hypovolemia are in use, also as i.v. infusions, since many years and show no toxic side effects. The dose recommendations known from such other medical uses specify an upper limit due to physical limits only. Solutions of "6% Hydroxyethyl Starch 130/0.4" in 0.9% sodium chloride can be administered repetitively over several days. Hence the patient can be provided with continued infusions of HES to treat his cancer and inhibit the growth rate of the tumor.

It is a preferred embodiment according to the invention that the administration of the substance HES or a pharmaceutical composition comprising HES is repeated according to the requirements of the patient.

In another preferred embodiment the therapeutically effective substance according to the invention is administered continuously.

Preferably, the hydroxyethyl starch is administered together with a suitable carrier, and/or a suitable diluent, such as preferably a sterile solution for i.v., i.m., i.p. or s.c. application. It is further preferred that the route of administration involves a ready-to-use liquid solution of the HES.

It is also preferred that the HES according to the invention is contained in a pharmaceutically acceptable device. It is also preferred that the HES is provided as an aqueous solution. It is further preferred that the aqueous solution is provided in a pharmaceutically acceptable container. Such a device may for example be in form of a syringe, bottle or a bag. The person skilled in the art will be able to select a suitable material. For example, a bottle may be made of glass or plastic materials and a bag may be made from plastic materials suitable and/or authorized for the containment of drugs. Preferably such an aqueous solution will be provided in a pharmaceutically acceptable bag suitable for the containment of drugs and/or the i.v. administration to patients. It is especially preferred that such a solution is provided in a plastic bag, such as for example the freeflex bag.

The term "therapeutically effective amount", as used herein, preferably refers to an amount of the hydroxyethyl starch as defined herein, or the pharmaceutical composition according to the present invention that (a) reduces or inhibits tumor growth rate, (b) treats the cancer, or (c) attenuates, ameliorates, or eliminates the cancer. It is also to be understood that the therapeutically active amount prevents the spread of cancer (metastasis), or prevents the generation of cancer or reduces tumor burden. More preferably, the term refers to the amount of the pharmaceutical composition comprising hydroxyethyl starch as the only active ingredient as defined herein, according to the present invention that (a) reduces or inhibits tumor growth rate, (b) treats the cancer, or (c) attenuates, ameliorates, or eliminates the cancer.

The term "subject", as used herein, relates to animals and, preferably, to mammals. More preferably, the subject is a rodent such as a mouse or a rat. Even more preferably, the subject is a primate. Most preferably, the subject is a human. According to the invention it is understood that the term "subject" also relates to an individual suffering from cancer or an individual in need of cancer treatment. In a preferred embodiment of the invention the term "subject" describes a cancer patient.

According to the invention the term "carcinoma" describes cancer types that arise in epithelia tissue, in the skin or in tissues that line or cover internal organs. According to the invention the cancer is preferably selected from the group consisting of skin, lung, colon, pancreatic and epithelial, squamous and basal cell carcinomas, melanomas, papillomas, and adenomas. Most preferably the cancer is selected from the group consisting of breast carcinoma, prostate carcinoma, lung carcinoma and renal carcinoma.

According to the invention the term "sarcoma" describes cancer types that usually arise from bone, cartilage, fat, muscle, blood vessels, or other connective or supportive tissue. According to the invention the cancer is preferably selected from the group consisting of bone cancer, soft tissue cancers, osteosarcoma, synovialsarcoma, liposarcoma, angiosarcoma, rhabdosarcoma, chondrosarcoma and fibrosarcoma.

According to the invention the term cancer also comprises cancer types that arise in the tissues of the brain and spinal cord. Preferably the cancer is selected from the group consisting of brain and spinal cord tumors, gliomas, meningiomas, pituitary adenomas, vestibular schwannomas, primary CNS lymphomas, and primitive neuroectodermal

tumors.

The term "hydroxyethyl starch" or "hydroxy ethylated starch" encompasses various hydroxyl-ethylated starches, as will be described in more detail below. These hydroxyethyl starches may be further substituted.

Hydroxyethyl starch is an ether derivative of partially hydrolyzed natural starches, in which hydroxyl groups in the starch are suitably hydroxyethylated.

The current invention comprises a new medical use of hydroxyethylated starches (HES) that are substituted with an ethyl residue which carries a hydroxy function.

Starch is a polysaccharide of the formula $(C_6H_{10}O_5)_n$ which is composed substantially of alpha-D-glucose units, coupled via glycoside linkages. Generally speaking, starch consists substantially of amylose and amylopectin. Amylose is composed of linear chains in which the glucose units are linked via alpha- 1,4-glycosidic linkages. Amylopectin has a highly branched structure, with alpha-1,4-glycosidic linkages and alpha-1,6-glycosidic linkages.

Natural starches from which hydroxyalkyl starches may be prepared include cereal starches, grain legume starches and potato starches. Cereal starches include rice starches, wheat starches such as einkorn starches, spelt starches, soft wheat starches, emmer starches, durum wheat starches or kamut starches, maize starches, rye starches, oat starches, barley starches, triticale starches and millet starches such as sorghum starches or teff starches. Grain legume starches include bean starches, pea starches, lentil starches and lupine starches. Preferred natural starches from which hydroxyalkyl starches are prepared have a high content of amylopectin relative to amylose. The amylopectin content of these starches is, for example, at least 70% by weight, preferably at least 75% by weight, more preferably at least 80% by weight, more preferably at least 85% by weight, more preferably at least 90% by weight, such as up to 95% by weight, up to 96% by weight, up to 97% by weight, up to 98% by weight or up to 99% by weight or up to 100% by weight. Natural starches having an especially high amylopectin content are, for example, suitable potato starches such as waxy potato starches, which are preferably extracted from substantially amylose-free potatoes, which are either traditionally cultivated, e.g. the natural variety Eliane, or genetically modified amylopectin potato varieties, and starches from waxy varieties of cereals such as waxy maize or waxy rice.

Generally, hydroxyalkyl starch is prepared by breaking starch grains and cleaving the macromolecules to obtain molecules having a desired size. Cleaving can be carried out, for example, by enzymatic degradation, as for example using alpha-amylase and/or beta-amylase, and/or by means of acidic hydrolysis. Purification of the desired fractions can be accomplished, for example, by means of ultrafiltration, using membranes having a suitable cut-off limit, which allow the separation, for example, of low-molecular by-products having a molecular weight of up to 5000 Da or up to 1000 Da. Two or more cleaving stages can be carried out in series, with the possibility in each stage of using the same or different cleaving technologies. After each cleaving stage, the product obtained can be purified. The product ultimately obtained can be isolated, as for example by freeze-drying.

On the basis of the starch fractions thus obtained, hydroxyalkyl starch is prepared by etherification of hydroxyl groups. In general, all reactions known from the etherification of low-molecular alcohols may be contemplated, such as reactions without catalyst or with basic catalysts. The preferred methods in technical processes include the Michael addition of activated olefins, the Williams synthesis with nucleophilic substitution of compounds containing aliphatic halogen, or the reaction with oxiranes, also known as epoxides.

Concerning the preparation of hydroxyethyl starch, reference is made, for example, to Sommermeyer et al., Chromatographia, 25, 1988, pp. 167-168; C. Jungheinrich et al., Clin. Pharmacokin., 44 (7), 2005, pp. 681-699; J.-M. Mishler IV, Pharmacology of hydroxyethyl starches, Oxford Medical Publications, 2002, pp. 1-30.

According to the present invention, the hydroxyethyl starch (HES), preferably has a structure according to the following formula (III)

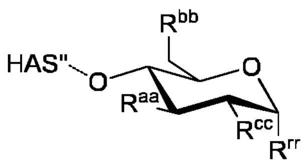

(III)

wherein $R^{aa}$, $R^{bb}$ and $R^{cc}$ are independently of each other selected from the group consisting of -O-HES", and $-[O-CH_2-CH_2]_s-OH$, wherein s is in the range of from 0 to 4 and wherein HAS", is the remainder of the hydroxyethyl starch and is abbreviated with HES". Residue $R^{rr}$ is either -O-HES" or, in case the formula (III) shows the terminal saccharide unit of HES, $R^{rr}$ is -OH.

As a polymer, and owing to the preparation processes, hydroxyethyl starch is a polydisperse compound in which the individual hydroxyethyl starch molecules may differ with respect to the degree of polymerization, the number

and the pattern of the branching sites, and the substitution pattern, i.e. the number and/or sites of the hydroxyethyl groups. Therefore, hydroxyethyl starch is usually characterized by statistically averaged parameters. These are, generally, the average molecular weight and parameters which characterize the substitution pattern. The latter parameters are typically identified as degree of substitution (DS), molecular substitution (MS) and C2/C6 ratio, i.e. the ratio of the number of anhydroglucose units substituted in C2 position to the number of anhydroglucose units substituted in C6 position, or the ratio of Mw relative to Mn (Mw/Mn), which is usually referred to as PDI (polydispersity index) and characterizes the spread of the molecular weight distribution.

Hydroxyethyl starch may be substituted with hydroxyethyl groups not only at the C2 and C6 sites, but also at the C3 site, but this information is usually omitted when referring to a specific type of HES.

The second parameter specifying a HES usually refers to the degree of molecular substitution MS and the third parameter either refers to the ratio of substitutions at C2 versus substitutions at C6 (C2/C6 ratio) or to the PDI.

Generally speaking, there are two ways of statistically describing the average molecular weight of hydroxyethyl starch. The first parameter is the number-average molecular weight, commonly referred to as Mn or $M_n$; the second parameter is the weight-average molecular weight, commonly referred to as Mw or $M_w$.

The molecular weight can be determined, for example, by means of gel permeation chromatography with multiple-angle light-scattering detection (GPC/MALLS/RI). Reference is made, for example, to W.-M. Kulicke et al., Starch, 45 (12), 1993, pp. 445-450. Alternatively, the molecular weight can be determined using flow-FFF/MALLS, as for example in accordance with European Pharmacopoeia 7.0, 01/2011:1785, p. 984 or else by B. Wittgren et al., Int. J. Polym. Anal. Charact. 7 (1-2), 2002, pp. 19-40.

In this context the number average molecular weight is defined by equation 1:

$$\overline{M}_n = \frac{\sum_i n_i \cdot M_i}{\sum_i n_i}$$

(1)

in which $n_i$ is the number of hydroxyethyl starch molecules of species i having molar mass $M_i$. $\overline{M}_n$ indicates that this is an average value, but the line is typically omitted.

The weight average molecular weight $M_w$ is defined by the following equation:

$$\overline{M}_w = \frac{\sum_i n_i \cdot M_i^2}{\sum_i n_i M_i}$$

(2)

in which $n_i$ is the number of hydroxyethyl starch molecules of species i having molar mass $M_i$. $\overline{M}_w$ indicates that this is an average value, but the line is typically omitted.

In the context of the present description the term "mean molecular weight" refers to the weight determined by the MALLS (multiple angle laser light scattering)-GPC method.

Hydroxyethyl starches according to the invention have a mean molecular weight (Mw or MW) varying from as low as about 20 kDa to mean molecular weights up to 1300 kDA.

The ratio of Mw relative to the Mn (Mw/Mn), which is usually referred to as PDI, polydispersity index, is a parameter characterizing the spread of the molecular weight distribution. The closer this parameter is to the value 1, the less dispers the molecular weight distribution is.

According to the invention typical PDI values are in the range of from 4.0 to 1.1.

The substitution pattern can be determined quantitatively, at least partially, using $^1$H NMR or by a more elaborate method, by means of high-resolution $^{13}$C NMR. Reference is made to Y. M. Liu et al., Chin. Chem. Lett. 13 (11), 2002, pp. 1097-1099, and to W.-M. Kulicke et al., Starch, 45 (12), 1993, pp. 445-450. In general there are three customary

parameters which describe the degree of substitution of hydroxyethyl starch.

The first parameter, which is identified as "DS" (degree of substitution), describes the ratio of the number of substituted anhydroglucose units to the total number of all the anhydroglucose units. In view of this definition, the theoretical maximum value of DS is 1.0.

The parameter DS can be determined, for example, in accordance with W. Banks et al., Br. J. Pharmac., 47, 1973, pp. 172-178, O. Larm et al., Starch, 33 (7), 1981, pp. 240-244, or Sommermeyer et al., Starch, 44 (6), 1992, pp. 215-218.

The second parameter, which is typically identified as "MS" (molecular substitution), describes the ratio of the number of hydroxyethyl residues (in mol) which have been added by hydroxyethylation to the glucose molecules of the starch macromolecule, relative to the number of glucose monomers in the molecule.

Assuming that the ethylation results in the addition of a single ethyl unit per hydroxy function, the degree of molar substitution indicates what proportion of the three hydroxy units the glucose units on the starch molecule have been substituted or replaced by hydroxyethyl units. Herein a substitution degree of 1 equals a 100% of substitution of one of the three free hydroxy groups. Hence theoretically the range of substitution could vary from 0.1 to 3, wherein three indicated that all three hydroxy units would be 100% substituted. There is a number of different types of HES in the market, and their substitution degrees vary from 0.3 to 2.

The parameter MS may be determined in accordance with Ying-Che Lee et al., Anal. Chem. 55, 1983, pp. 334-338; or K. L. Hodges et al., Anal. Chem. 51, 1979, p. 2171. According to these methods, a known amount of the hydroxyethyl starch is subjected to an ether cleavage in xylene, with addition of adipic acid and hydriodic acid. The amount of iodoalkane released is subsequently determined by means of gas chromatography, using toluene as an internal standard and iodoalkane calibration solutions as external standards.

The third parameter, which is identified as the C2/C6 ratio, describes the ratio of the number of anhydroglucose units substituted in C2 position to the number of anhydroglucose units substituted in C6 position. During the preparation of the hydroxyethyl starch, the C2/C6 ratio can be influenced via the amount of base used for the hydroxyethylation reaction. Generally speaking, the higher the concentration of base, the greater the number of hydroxyl groups which are hydroxyethylated in C6 position.

The parameter C2/C6 can be determined, for example, in accordance with Sommermeyer et al., Krankenhauspharmazie 8 (8), 1987, pp. 271-278, especially page 273.

Various types of hydroxyethyl starch, therefore, are usually described by a statement of their average molecular weight, expressed in kDa, their degree of molar substitution (MS), and their degree of branching (C2/C6), or by an indication of their polydispersity (Mw/Mn).

The present invention provides a fundamentally new active therapeutic agent for the treatment of cancer, which avoids the problematic side effects associated with the administration of other cancer therapeutics, especially of chemotherapeutics, such as cytostatica. In particular, the toxic side effects associated with the administration of chemotherapeutics, indicated by a loss of body weight and cachexia, as a response to the drastic treatment with cell toxic agents, can be avoided when treating the cancer by way of reducing the tumor growth rates with hydroxyethylated starch (HES).

The present invention relates to HES for the treatment of cancer. In a further embodiment, the invention relates to a pharmaceutical composition comprising HES according to the invention for the treatment of cancer. In a further embodiment invention relates to methods of treatment of cancer comprising the administration of HES according to the invention.

It was unexpectedly found that the administration of HES to mammals that were inoculated with tumor cell lines, i.e. were suffering from cancer, alleviated their symptoms and resulted in a significant growth reduction of the tumor, whilst not adversely affecting their health or body weight. The therapeutic effect was observed for various HES solutions. Table 1 below shows a number of tested HES solutions, with specific parameters given for the HES type used in experimental settings with inoculated mice:

Table 1:

| "Name of HES type" exemplified by Mw/Ms/PDI | Cell line | Figure | Example |
|---|---|---|---|
| "HES 70/0.5" 66/0.57/2.3 | MT-3 human breast carcinoma | Fig 7, Fig 8 | 5 |
| "HES 100/0.1/2.0" | MT-3 human breast carcinoma | not displayed | - |
| "HES 100/0.7/1.3" 103/0.7/1.3 | MT-3 human breast carcinoma | Fig 11, Fig 12 | 5 |
| "Voluven 10% 130/0.4" 105/0.4 | MT-3 human breast carcinoma | Fig 4, Fig 2 | 1, 2 |
| "HES 100/1.0/1.3" 84/1.0/1.3 | MT-3 human breast carcinoma | Fig 4, Fig 2 | 1,2 |
| "HES 100/1.0/1.3" 78/1.0/1.4 | MT-3 human breast carcinoma | Fig 7, Fig 8 | 5 |

(continued)

| "Name of HES type" exemplified by Mw/Ms/PDI | Cell line | Figure | Example |
|---|---|---|---|
| "HES 130/0.4" 105/0.4 | MT-3 human breast carcinoma | Fig 7, Fig 8 | 5 |
| (dissolved in saline) | | | |
| "HES 200/0.5" 195/0.46 | MT-3 human breast carcinoma | Fig 9, Fig 10 | 5 |
| "HES 450/0.7" 420/0.7 | MT-3 human breast carcinoma | Fig 7, Fig 8 | 5 |
| "HES 700/0.5" 618/0.5/2.2 | MT-3 human breast carcinoma | Fig 9, Fig 10 | 5 |
| "HES 700/0.7" 644/0.7/1.9 | MT-3 human breast carcinoma | Fig 9, Fig 10 | 5 |
| "HES 700/1.3" 728/1.3/1.6 | MT-3 human breast carcinoma | Fig 9, Fig 10 | 5 |
| "HES 900/0.4" 929/0.4/3.0 | MT-3 human breast carcinoma | Fig 11, Fig 12 | 5 |
| "HES 900/0.7" 1034/0.67/3.0 | MT-3 human breast carcinoma | Fig 11, Fig 12 | 5 |
| "HES 1300/0.7" 1406/0.7/4.4 | MT-3 human breast carcinoma | Fig 11, Fig 12 | 5 |
| "Voluven 10% 130/0.4" 105/0.4 | PC3 xenograft prostate | Fig 5 | 4 |

All tested starches showed a therapeutic effect resulting in a reduced tumor growth rate, compared to the growth rate of untreated subjects, or subjects treated with saline control only.

Without wanting to be bound to any theory, it may be that the hydroxyethylated starches affect the angiogenesis of the tumor and inhibit its further growth and the progression of cancer. Angiogenesis plays a critical role in the growth and spread of cancer. Tumors are depending from a growing network of capillaries, providing oxygen and nutrients. To grow a tumor requires the new formation of blood vessels. Without the ability to generate new blood vessels for provision of nutrients the non-angiogenetic neoplasia remain of a clinically irrelevant size and do not cause symptoms, the cancer cells cannot invade nearby tissue, to move throughout the body, and to form new colonies of cancer cells, called metastases. By inhibiting angiogenesis tumor dormancy is achieved. Hence anti-angiogenetic therapies try to block or reduce the blood circulation of the tumor by blocking or reducing its blood vessel building. It is possible that the polymeric substance of HES blocks the newly formed blood vessels and thereby hinders the tumor to grow and the cancer to progress.

In a preferred embodiment the present invention relates to a hydroxyethyl starch and a pharmaceutical composition comprising a HES according to the invention, for the reduction of tumor size or growth rate thereof.

Advantageously, it has been shown in the studies carried out in the context of the present invention that the hydroxyethyl starch or the pharmaceutical composition comprising said hydroxyethyl starch is not toxic and triggers hardly any side effects when given intravenously, which is of great advantage when compared to a cytotoxic agent. It does not result in a body weight reduction such as routinely used cytotoxic agents, as, for example, docetaxel (Taxotere®). Hence wherein the max. dosage of a conventional cytotoxic agent is limited severely by its toxic side effects, a patient can receive repeated doses and continuos infusion of hydroxyethyl starches on a daily basis.

It is preferred that the hydroxyethyl starch has a mean molecular weight MW of above 20 kDa, preferably above 40 kDa, and even more preferably a MW greater than 65 kDa. Preferably the MW is also not higher than 1300 kDa. More preferably the MW is in the range of from 75 to 1200 kDa, and more preferably in the range of from 90 to 800 kDa.

In one embodiment, the hydroxyethyl starch (HES) according to the invention has a molar substitution degree MS of the HES in the range of from 0.1 to 1.5. Preferred embodiments comprise particular ranges of molar substitutions values of 0.15 to 1.5, 0.2 to 1.5, 0.3 to 1.5, 0.4 to 1.5, 0.5 to 1.5, 0.6 to 1.5, 0.7 to 1.5, 0.75 to 1.5, more preferably in the range of 0.1 to 1.3, 0.1 to 1.0, 0.1 to 0.8, 0.1 to 0.6, and 0.1 to 0.5 and also preferably in the range of from 0.90 to 1.4, such as 0.90, 0.95, 1.0, 1.05, 1.1, 1.15, 1.2, 1.25, 1.3, 1.35 or 1.4. A particularly preferred range is from 0.1 to 1.0, more preferably from 0.1 to 0.6, more preferably from 0.25 to 0.55.

According to an especially preferred embodiment, the hydroxyethyl starch derivative has a mean molecular weight MW in the range of from 80 to 1200 kDa and a MS in the range of from 0.1 to 1.5. Preferred embodiments comprise particular ranges of molar substitutions values of 0.15 to 1.45, 0.3 to 1.45, 0.45 to 1.45, 0.6 to 1.45, 0.7 to 1.45, 0.75 to 1.45, more preferably in the range of 0.1 to 0.5 and preferably in the range of from 0.90 to 1.4, such as 0.90, 0.95, 1.0, 1.05, 1.1, 1.15, 1.2, 1.25, 1.3, 1.35 or 1.4, more preferably a molar substitution MS in the range of from 0.1 to 1.30, or 0.1 to 0.5.

In an especially preferred embodiment, the hydroxyethyl starch derivative has a mean molecular weight MW in the range of from 30 to 700 kDa and a molar substitution in the range of from 0.1 to 0.7; more preferably a mean

molecular weight MW in the range of from 80 to 700 kDa and a MS in the range of from 0.1 to 0.7.

In one embodiment the C2/C6 ratio of HES, is in the range of from 0.5 to 20, more preferably in the range of from 2 to 20, 18, 2 to 17, 2 to 14, 2 to 12, 2 to 10, 2 to 8, 2 to 6, 2 to 5, or 2 to 4, . In another preferred embodiment said C2/C6 substitution is in the range of from 4 to 12, 6 to 12, 7 to 12, or preferably in the range of from 7 to 10, more preferably in the range from 8 to 9. In another preferred embodiment said C2/C6 substitution is in the range of from 4 to 6, more preferably is 5.7.

In a preferred embodiment, the polydispersion index PDI is in the range of from 1.1 to 4.0, more preferably in the range of from 1.1 to 3.5, 1.1 to 3, 1.1 to 2.5, 1.1 to 2, 1.1 to 1.5, 1.1 to 1.4, 1.1 to 1.3 and 1.1 to 1.2. In another preferred embodiment the PDI is in the range of from 1.2 to 4, 1.35 to 4, 1.5 to 4, 1.7 to 4, 1.8 to 4, 1.9 to 4, 2 to 4, 2.5 to 4 or 2 to 4, or 1.4 to 3.0.

All of these ranges are considered to comprise values that differ from the precise numbers given by about a tenth of their numeric value.

Preferably, the hydroxyethyl starch, as described above, has a mean molecular weight MW (weight mean) above the renal threshold.

In another preferred embodiment the hydroxyethyl starch according to the invention, as described above, has a mean molecular weight MW (weight mean) below the renal threshold.

The renal threshold is determined according to the method described by Waitzinger et al. (Clin. Drug Invest. 1998; 16: 151-160) and reviewed by Jungheinrich et al. (Clin. Pharmacokinet. 2006; 44(7): 681-699). Preferably, the renal threshold is denoted to mean a molecular weight MW equal to or higher than 40 kDa, or 45 kDa or 60 kDa or 65kDa.

In the following, hydroxyethyl starch structures are described in more detail, which comprise several different preferred embodiments of the described class of HES.

In one preferred embodiment the hydroxyethylated starch is a hydroxyethylated starch known under the name "HES 130/0.4". Despite the name "HES 130/0.4" this is a hydroxyethylstarch with a mean molecular weight of 105 kDa, according to the standard measurement and calibration method described in European Pharmacopoeia 7.0, 01/2011:1785, p.984, with a molar substitution degree in the range of 0.38--0.45, a mean molar substitution degree of 0.4. Its C2/C6 ratio is between 8.0 and 12.0. Its PDI is about 2, i.e. between 1.7 and 2.3. It is commercially available, for example as a 10% solution in 0.9% NaCl solution, under the registered trade name Voluven®. The difference between the value of the MW 130 of the publicly known specification of "HES 130/0.4" and the amended specification to HES 105/0.4 results from a change in the method calibration used for determining the Mw of HES. Whereas previously the determination was performed according to Sommermeyer et al. (Krankenhauspharmazie, 8, 1987, 08, p. 271-278), the amended value (Mw 105) has been determined according to the calibration as described in European Pharmacopoeia 7.0, 01/2011:1785, p.984. The difference in the method is the value of the light scattering value dn/dc, wherein in the Sommermeyer method a dn/dc value of 0.135 is used, this value changed to 0.147+/-0.001 in the "Pharmacopoeia method".

Another preferred embodiment according to the invention is a hydroxyethylated starch known as "HES 100/1.0/1.3". This is a hydroxyethylstarch with a mean molecular weight of 100 kDa, determined according to Sommermeyer et al.; and with a mean molecular weight of about 84 kDa (75-93 kDa), determined according to European Pharmacopoeia 7.0, 01/2011:1785, p.984; and a molar substitution degree of $1.0\pm0.05$. Its C2/C6 ratio is 5.0 - 6.0 or preferably 5.7 and the PDI is $1{,}3\pm0.1$.

Often the name indicated in parentheses such as "HES 200/0.5" refers to the old measurement, but it is explained herein, which Mw values will be generated if measured according to European Pharmacopeia (as cited before). The Mw values in the application (which are not part of the "name") refer to those determined according to European Pharmacopoeia 7.0, 01/2011:1785, p.984 with the calibration method defined therein using a dn/dc value of 0.147-0.149, unless specifically mentioned otherwise.

It is another especially preferred embodiment according to the invention wherein said "HES 100/1.0/1.3" is further specified by one or more of the properties listed in Table 2 specifying the batch of "HES 100/1.0/1.3" as used in the examples 1 and 2.

Table 2:

| Test parameter | Measured properties of "HES 100/1.0/1.3" used in Example 2 |
|---|---|
| Appearance | Solid |
| Colour | yellowish |
| Absorption 400 nm/1cm | 0.007 |
| Mw | 84 kDa +/- 8.4 kDa |
| Mw of the 10% smallest fraction | 31.700 Da |

(continued)

| Test parameter | Measured properties of "HES 100/1.0/1.3" used in Example 2 |
|---|---|
| Mw of the 10% largest fraction | 177.567 Da |
| Mn | 64.883 Da |
| Mw/Mn (PDI) | 1.29 |
| MS | 0.99 |
| C2/C6 | 5.7 |

In Example 5 another batch of a so called "HES 100/1.0/1.3" has been used, as defined in this table 3:

Table 3:

| Test parameter | Measured properties of "HES 100/1.0/1.3" used in Example 5 |
|---|---|
| Appearance | Solid |
| Colour | yellowish |
| Absorption 400 nm/1cm | 0.007 |
| Mw | 78,4 kDa |
| Mw of the 10% smallest fraction | 24.977 Da |
| Mw of the 10% largest fraction | 178.700 Da |
| Mn | 55.993 Da |
| Mw/Mn (PDI) | 1.40 |
| MS | 1.02 |
| C2/C6 | 5.6 |

Another embodiment is a hydroxyethylated starch known as "HES 70/0.4/1.8" for treatment of cancer. This is a hydroxyethylstarch with a mean molecular weight of 70 kDa, a molar substitution degree of 0.4 and a PDI of 1.8.

Another embodiment is a hydroxyethylated starch known as "HES 70/0.5" for treatment of cancer. This is a hydroxyethylstarch with a mean molecular weight of 70 kDa, a molar substitution degree of 0.5.

Another embodiment is a hydroxyethylated starch HES 100/0.1/2.0 for treatment of cancer. This is a hydroxyethylstarch with a mean molecular weight of 100 kDa, a molar substitution degree of 0.1 and a PDI of 2.0.

Another embodiment is a hydroxyethylated starch named as "HES 100/0.1/2.0" for treatment of cancer. This is a hydroxyethylstarch with a mean molecular weight of 130 kDa, a molar substitution degree of 0.1 and a PDI of 2.0.

Another embodiment is a hydroxyethylated starch known as "HES 100/0.7/1.3" for treatment of cancer. This is a hydroxyethylstarch with a mean molecular weight of 100 kDa, a molar substitution degree of 0.7 and a PDI of 1.3.

Another embodiment is a hydroxyethylated starch known as "HES 100/1.0/1.1" for treatment of cancer. This is a hydroxyethylstarch with a mean molecular weight of 100 kDa, a molar substitution degree of 1.0 and a PDI of 1.1.

Another embodiment is a hydroxyethylated starch known as "HES 150/0.7/1.3" for treatment of cancer. This is a hydroxyethylstarch with a mean molecular weight of 150 kDa, a molar substitution degree of 0.7 and a PDI of 1.3.

Another embodiment is a hydroxyethylated starch known as "HES 150/1.0/1.3" for treatment of cancer. This is a hydroxyethylstarch with a mean molecular weight of 150 kDa, a molar substitution degree of 1.0 and a PDI of 1.3.

Another embodiment is a hydroxyethylated starch known as "Viastarch" with a mean molecular weight of: Mw 150--300 kDa,a molar substitution degree of MS 0.40--0.50, further characterized by Mw of lowest 10% fraction >=25 kDa, Mw of highest 10% fraction <=2000 kDa, which may also be referred to as "HES 180/0.45", for treatment of cancer.

Another embodiment is a hydroxyethylated starch known as "HES 200/0.5" for treatment of cancer. This is a hydroxyethylstarch with a mean molecular weight of 200 kDa, further characterized by a Mw 170-290, and of a molar substitution degree of 0.43 to 0.55. This HES may be further characterized by Mw of lowest 10% fraction >15 kDa, and Mw of highest 10% fraction <600 kDa.

Another embodiment is a hydroxyethylated starch known as "Pentastarch" with a mean molecular weight of: Mw 200-300 kDa, and a MS of 0.40-0.50; further characterized by Mw of lowest 10% fraction >=15 kDa, Mw of highest 10% fraction <=1500 kDa, which can be referred to as "HES 250/0.45", for treatment of cancer.

Another embodiment is a hydroxyethylated starch known as "HES 300/1.0/1.3" for treatment of cancer. This is a hydroxyethylstarch with a mean molecular weight of 250+/-17kDa (or 300 kDa according to Sommermeyer et al.), a molar substitution degree of 1.0+/-0.05 and a PDI of 1.3+/-0.1.

Another embodiment is a hydroxyethylated starch with a mean molecular weight of 300 kDa, a substitution degree Ds of below 0.4 as described in WO 00/48637, for treatment of cancer.

Another embodiment is a hydroxyethylated starch known as "HES 450/0.7" for treatment of cancer. This is a hydroxyethylstarch with a mean molecular weight of 450 kDa (Mw 400--500 kDa), which may be further specified by a Mw of lowest 10% fraction >=25 kDa, and a Mw of highest 10% fraction <=3000 kDa; and a molar substitution degree of 0.7 (MS 0.65--0.75).

Another embodiment is a hydroxyethylated starch with a mean molecular weight of 500 kDa according to the method referred to under Sommermeyer et al. and a molar substitution degree of 0.28 and a C2/C6 ratio of 8.7 described in and according to US patent 5,502,043 "Use of hydroxyethyl starch for improvement of microcirculation" to Weidler et al., in example 3, for the treatment of cancer.

Another embodiment is a hydroxyethylated starch with a mean molecular weight of 500 kDa and a molar substitution degree MS between 0.25 and 0.5 and a C2/C6 ratio of 2 to below 8 described in and according to European patent EP1732953B (claim 1), for the treatment of cancer.

Another embodiment is a hydroxyethylated starch with a mean molecular weight of 600 kDa and a molar substitution degree of 0.5 described in and according to European patent EP0402724B by Fresenius AG for the treatment of cancer.

Another embodiment is a hydroxyethylated starch known as "HES 700/0.5/2.5" for treatment of cancer. This is a hydroxyethylstarch with a mean molecular weight of 600+/- 40 kDa (or 700 kDa according to Sommermeyer et al.), a molar substitution degree of 0.5+/-0.05 and a PDI of 2.5.

Another embodiment is a hydroxyethylated starch known as "Hetastarch", with a mean molecular weight of: Mw 550--800 kDa, MS 0.70--0.80, Mw of lowest 10% fraction >=13 kDa, Mw of highest 10% fraction <=4000 kDa; which can be described as "HES 700/0.7" for treatment of cancer.

Another embodiment is a hydroxyethylated starch known as "HES 700/0.7/2.0" for treatment of cancer. This is a hydroxyethylstarch with a mean molecular weight of 600+/- 40 kDa (or 700 kDa according to Sommermeyer et al.), a molar substitution degree of 0.7+/-0.05and a PDI of 2.0.

Another embodiment is a hydroxyethylated starch known as "HES 700/1.0/1.5" for treatment of cancer. This is a hydroxyethylstarch with a mean molecular weight of 600+/- 40 kDa (or 700 kDa according to Sommermeyer et al.), a molar substitution degree of 1.0+/-0.05 and a PDI of 1.5.

Another embodiment is a hydroxyethylated starch known as "HES 700/1.3/1.5" for treatment of cancer. This is a hydroxyethylstarch with a mean molecular weight of 600+/-40 kDa (or 700 kDa according to Sommermeyer et al.), a molar substitution degree of 1.3+/-0.05 and a PDI of 1.6+/-0.1.

Another embodiment is a hydroxyethylated starch known as "HES 60/1.3/1.3" for treatment of cancer. This is a hydroxyethylstarch with a mean molecular weight of 50+/-5 kDa (or 60 kDa according to Sommermeyer et al.), a molar substitution degree of 1.3+/-0.05 and a PDI of 1.3+/-0.1.

Another embodiment is a hydroxyethylated starch of a mean molecular weight Mw of 1000 kDa and a substitution degree Ds between 4 and 10 for, as described in US patent 6,680,305 for treatment of cancer.

Another embodiment is a hydroxyethylated starch known as "HES 70000", also referred to as "HES 70/0.55" with a mean molecular weight Mw of 60 - 80 kDa for treatment of cancer. Preferably it has a MS of 0.55-0.61. Preferably it has a PDI of 2.3 +/-0.1.

Another embodiment is a hydroxyethylated starch known of a mean molecular weight Mw of 70 kDa and a C2/C6 ratio between 2 to 8 for treatment of cancer as described in and according to A.N.Belder and B.Norman in Carbohydrate Research, Vol 10, 1969, p. 391-394.

The method of treating a subject suffering from a tumor with a HES according to the invention is one preferred embodiment of the invention. It is preferred that the method comprises a step of administering a therapeutically effective amount of said HES to said subject.

Another preferred embodiment is a pharmaceutical composition comprising HES according to the invention described herein, wherein HES is itself therapeutically active in reducing the tumor growth rate. It is preferred that such HES is the only therapeutically active ingredient therein.

It is a preferred embodiment wherein the therapeutic activity of HES results in an inhibitory effect on the proliferating activity of the tumor cells, wherein HES reduces the proliferation rate of tumor cells. This is based on the observations which were made when the tumor tissue of treated and untreated mice was compared.

After the mice were sacrificed in studies as described, for example, in Example 5, the tumor tissue was excised from the corpse and an analysis was performed to compare the effect of HES administered to tumor mice on intratumoral necrosis, number of mitotic figures, Ki67-index and CD31 staining intensity in a murine tumor model.

48 different tumor preparations have been analysed with a standard test based on the staining of KI positive

cells. In addition a "CD31 staining" has been performed to determine the density of the tumor stroma. Due to technical difficulties only a limited set of samples could be analysed with the latter staining, but in all samples tested the density appeared to be similar.

In summary, comparison of tumor tissues of group A, from mice treated with saline only, with group V, from mice treated with Voluven 10%, revealed a decreased number of mitotic figures in group V, which is indicative for a decreased proliferative activity of tumor cells in group V. Group V tumors also contained slightly more necrosis than group A tumors. No obvious differences between group A and V were identified using a semiquantitative Ki67-index to analyze the percentage of proliferative tumor cells and a CD31-staining to analyze the density of intratumoral vessels.

Hence, it could be shown that HES solutions have a direct effect on the proliferation rate of tumor cells, which is reduced in cells from HES treated tumor tissue, whereas the treatment with HES solutions does not affect normally proliferating cells in healthy tissues.

Therefore, without being bound to a theory, we assume that HES treatment results in reducing the tumor growth rate by inhibition or deceleration of the cell proliferation rate of tumor cells, which is caused by a reduced number of tumor cells in mitosis (which refers to a reduced number of doubling tumor cells). The data indicate that the mitotic activity of the tumor cells is reduced.

It is an embodiment of the invention wherein HES is therapeutically active in reducing the tumor growth rate by reducing or inhibiting the proliferation rate or arresting the mitotic cycle of tumor cells or cells proliferating without physiological control. It has to be stressed that HES does not reduce the proliferation rate of non tumor cells or normally proliferating cells.

It is an embodiment of the invention wherein HES is therapeutically active in reducing the tumour growth rate by arresting tumor cells in the mitotic cycle.

In a preferred embodiment according to the invention the cancer is selected from the group of solid tumors arising in solid tissues or organs. It is preferred that the group of solid tumors does not contain head and neck tumors.

It is also preferred that the group of tumors does not contain ovary or bladder cancer.

In a preferred embodiment according to the invention the cancer is selected from the group consisting of biliary cancer, breast cancer, cervical cancer, colorectal cancer, gastrointestinal cancer, malignant melanoma, mesothelioma, pancreatic cancer, prostate cancer, sarcoma, thyroid cancer, non-small cell lung cancer, and small cell lung cancer.

It is especially preferred that the cancer is selected from the group consisting of breast cancer, colorectal cancer, prostate cancer, and small cell and non-small cell lung cancer.

In one embodiment of the invention the cancer is breast cancer.

In one embodiment the cancer is selected from the group consisting of colorectal cancer, gastrointestinal cancer and kidney cancer.

In one embodiment the cancer is selected from the group consisting of small cell lung cancer and non-small cell lung cancer.

In one embodiment the cancer is selected from the group consisting of prostate cancer, and renal cancer

In one embodiment the cancer is cervical cancer.

In one embodiment the cancer is selected from the group consisting of pancreatic cancer and biliary cancer.

In one embodiment the cancer is selected from the group consisting of sarcoma, mesothelioma and malignant melanoma.

In one embodiment according to the invention the tumor has been caused by a prostate cancer.

In another preferred embodiment according to the invention the tumor has been caused by a cancer selected from the group consisting of carcinoma. It is especially preferred that the tumor has been caused by a prostate carcinoma, a breast carcinoma, a lung carcinoma or a renal carcinoma.

It is especially preferred that said carcinoma are selected from the group consisting of skin, lung, colon, melanoma and ovarian carcinoma.

In another preferred embodiment of the invention the tumor is associated with a cancer selected from the group consisting of sarcoma.

In another preferred embodiment of the invention the tumor is associated with a cancer selected from the group consisting of cancer types that arise in the tissues of the brain and spinal cord.

It is especially preferred that the tumor which is treated with HES is defined as a "fast growing tumor".

The term "fast growing tumor" is characterised as showing a tumor volume doubling time in a mouse model of less or equal to 4 days. The tumor volume doubling time (DT) is defined as the time interval (in days) required for a group of mice to reach a median RTV (relative tumor volume) of 200% of the initial tumor volume (normally from 100-200 $mm^3$). The tumor volume doubling time is an established parameter for the quantification of the tumor proliferation. Tumor volume doubling time for a given tumor may vary to some extent between experiments, which is why a tumor is classified a fast growing when it has a median doubling time (in a mouse model) beteen 1 and 4 (+/-0.4) days. A tumor is classified as slow growing when it has a median doubling time (in a mouse model) of more than 6 (+/-0.6) days. Examples of fast growing tumor xenografts are LXFE 397 and RXF 2178.

It is especially preferred that the tumor is selected from the group of fast growing tumors of solid tumors arising in solid tissues or organs.

It is more preferred that the tumor is selected from the groups of fast growing carcinomas.

It is even more preferred that the fast growing tumor is derived from a cancer selected from the group consisting of biliary cancer, breast cancer, cervical cancer, colorectal cancer, gastrointestinal cancer, malignant melanoma, mesothelioma, pancreatic cancer, prostate cancer, renal cancer, sarcoma, thyroid cancer, non-small cell lung cancer, and small cell lung cancer.

In one embodiment the therapeutic effect of HES, or the treatment is further characterized as a treatment of cancer wherein no or significantly less toxic side effects for the treated patient are shown than when given cytostatica. The effect of HES treatment therefore is reducing the tumor growth rate, whilst not causing a decreased general health status.

Another preferred embodiment of the present invention pertains to the use of the hydroxyethyl starch, or the pharmaceutical composition, according to the present invention for the manufacture of a medicament for the treatment of cancer, wherein the hydroxyethyl starch is the therapeutically active ingredient.

Finally, the present invention also relates to a method of treatment of a subject in need thereof comprising a step of administering hydroxyethyl starch, as the therapeutically active ingredient, to a subject in need thereof, resulting in stop or inhibition of the progression of cancer, preferably resulting in a reduced tumor size or reduced growth rate of the tumor. It is preferred that the method comprises a step of administering a therapeutically effective amount of said HES to said subject. Treatment methods according to the invention may be targeted to all cancer types mentioned herein, and the HES administered may comprise all types of HES disclosed herein.

The following especially preferred embodiments are described:

1. A hydroxyethyl starch (HES), as therapeutically active compound, for treatment of cancer, characterized as a cancer comprising a growing tumor, wherein the administration of HES results in reduced tumor growth rates, or a hydroxyethyl starch (HES), as therapeutically active compound, for reducing tumor growth rates.
2. A hydroxyethyl starch (HES) according to embodiment 1 wherein the hydroxyethyl starch comprises at least one structural unit according to the following formula (I)

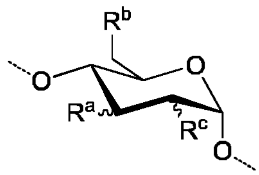

(I)

wherein $R^a$, $R^b$ and $R^c$ are independently of each other selected from the group consisting of -O-HES", $-[O-(CR^wR^x)-(CR^yR^z)]_x-OH$, $-[O-(CR^wR^x)-(CR^yR^z)]_y-XH$, wherein $R^w$, $R^x$, $R^y$ and $R^z$ are independently of each other selected from the group consisting of hydrogen and ethyl,
y is an integer in the range of from 0 to 20, preferably in the range of from 0 to 4, and x is an integer in the range of from 0 to 20, preferably in the range of from 0 to 4, and wherein at least one of $R^a$, $R^b$ and $R^c$ is $-[O-(CR^wR^x)-(CR^yR^z)]_y-XH$ and wherein X is selected from the group consisting of -S-, and -O-.
3. A hydroxyethyl starch (HES) according to embodiment 1, wherein the hydroxyethyl starch has a mean molecular weight Mw between 20 and 1300 kDa.
4. A hydroxyethyl starch (HES) according to embodiment 1, wherein the hydroxyethyl starch has a mean molecular weight Mw between 40 and 1300 kDa.
5. A hydroxyethyl starch (HES) according to embodiment 1, wherein the hydroxyalkyl starch has a mean molecular weight Mw between 65 and 1300 kDa.
6. A hydroxyethyl starch (HES) according to embodiment 1, wherein the hydroxyethyl starch has a mean molecular weight Mw between 70 and 1200 kDa.
7. A hydroxyethyl starch (HES) according to embodiment 1, wherein the hydroxyethyl starch has a mean molecular weight Mw between 75 and 800 kDa.
8. A hydroxyethyl starch (HES) according to embodiment 1, wherein the hydroxyethyl starch has a mean molecular weight Mw between 90 and 800 kDa.
9. A hydroxyethyl starch (HES) according to any of the embodiments above, wherein the hydroxyethyl starch has a mean molecular weight Mw between 100 and 700 kDa.
10. A hydroxyethyl starch (HES) according to any of the embodiments above, wherein the hydroxyethyl starch has

a mean molecular weight Mw between 100 and 110 kDa.

11. A hydroxyethyl starch (HES) according to any of the embodiments above, wherein the hydroxyethyl starch has a mean molecular weight Mw above the renal threshold.

12. A hydroxyethyl starch (HES) according to embodiment 1 or 2, wherein the hydroxyethyl starch has a mean molecular weight Mw below the renal threshold.

13. A hydroxyethyl starch (HES) according to any of the embodiments above, wherein the hydroxyethyl starch has a molecular substitution MS between 0.1 and 1.5.

14. A hydroxyethyl starch (HES) according to any of the embodiments above, wherein the hydroxyethyl starch has a molecular substitution MS between 0.1 and 1.3.

15. A hydroxyethyl starch (HES) according to any of the embodiments above, wherein the hydroxyethyl starch has a molecular substitution MS between 0.1 and 1.1.

16. A hydroxyethyl starch (HES) according to any of the embodiments above, wherein the hydroxyethyl starch has a molecular substitution MS between 0.1 and 0.9.

17. A hydroxyethyl starch (HES) according to any of the embodiments above, wherein the hydroxyethyl starch has a molecular substitution MS between 0.3 and 0.8.

18. A hydroxyethyl starch (HES) according to any of the embodiments above, wherein the hydroxyethyl starch has a molecular substitution MS between 0.3 and 0.7.

19. A hydroxyethyl starch (HES) according to embodiment 7 wherein the hydroxyethyl starch has a mean molecular weight between 80 and 230 kDa and a molecular substitution MS between 0.3 and 0.6.

20. A hydroxyethyl starch (HES) according to embodiment 10 wherein the hydroxyethyl starch has a mean molecular weight of 100 to 110 kDa and a molecular substitution MS between 0.3 and 0.5.

21. A hydroxyethyl starch (HES) according to embodiment 19 wherein the hydroxyethyl starch has a mean molecular weight between 150 and 200 kDa and a molecular substitution MS between 0.4 and 0.5.

22. A hydroxyethyl starch (HES) according to embodiment 21 wherein the hydroxyethyl starch has a mean molecular weight of 105 kDa and a molecular substitution MS of 0.4, preferably an MS of 0.42+/-0.05.

23. A hydroxyethyl starch (HES) according to embodiment 9 wherein the hydroxyethyl starch has a mean molecular weight between 400 and 700 kDa and a molecular substitution MS between 0.6 and 0.8.

25. A hydroxyethyl starch (HES) according to any of the embodiments above wherein the HES is provided as an aqueous solution and filled into a container suitable for clinical use, wherein a preferred container for clinical use is a bottle or a bag or any other container made of glass or types of plastic materials that are authorized for the containment of drugs.

26. A hydroxyethyl starch (HES) according to any of the embodiments above wherein the treatment is characterized as inhibiting tumor growth or reducing tumor growth rates or tumor size.

27. A hydroxyethyl starch according to any of embodiments 1 to 26, wherein the cancer is selected from the group consisting of solid tumors, which are tumors arising in solid tissues.

29. A hydroxyethyl starch according to any of embodiments 1 to 26, wherein the cancer is selected from the group consisting of cancer types arising in the skin or in tissues that line or cover internal organs, such as skin, lung, colon, pancreatic, ovarian, epithelial, squamous and basal cell carcinomas, melanomas, papillomas, and adenomas.

30. A hydroxyethyl starch according to any of embodiments 1 to 26, wherein the cancer is selected from the group consisting of bone cancer, soft tissue cancer, osteosarcoma, synovialsarcoma, chondrosarcoma, liposarcoma, angiosarcoma, rhabdhosarcoma and fibrosarcoma.

31. A hydroxyethyl starch according to any of embodiments 1 to 26, wherein the cancer is selected from the group consisting of brain and spinal cord tumors, gliomas, meningiomas, pituitary adenomas, vestibular schwannomas, primary CNS lymphomas, and neuroectodermal tumors.

32. A hydroxyethyl starch according to any of embodiments 1 to 26, wherein the cancer is selected from the group consisting of biliary cancer, bladder cancer, breast cancer, cervical cancer, colorectal cancer, gastrointestinal cancer, malignant melanoma, mesothelioma, non-small cell lung cancer, pancreatic cancer, prostate cancer, sarcoma and small cell lung cancer.

33. A hydroxyethyl starch according to embodiment 32, wherein the cancer is selected from the group consisting of bladder cancer, breast cancer, cervical cancer, colorectal cancer, prostate cancer, non-small cell lung cancer and small cell lung cancer.

34. A hydroxyethyl starch according to embodiment 32, wherein the cancer is selected from the group consisting of breast cancer, prostate cancer, non-small cell lung cancer and small cell lung cancer.

35. A hydroxyethyl starch according to any of the embodiments above, wherein the cancer is characterized as presenting a tumor type, characterized by a median doubling time of below 6 days, or more preferably of below or equal to 4 days.

36. A hydroxyethyl starch according to embodiment 35, wherein the doubling time is determined in a mouse model suitable to determine cancer growth rates, preferably in a mouse model as described in example 6.

37. A pharmaceutical composition comprising a hydroxyethyl starch (HES) according to any of the embodiments 1 to 26, wherein HES is the only therapeutically active component for the treatment of cancer, characterized as reducing the growth rate of tumors.
38. A pharmaceutical composition comprising a hydroxyethyl starch (HES) according to embodiment 37, characterized as being contained in a suitable container, preferably made of glass or plastic materials, preferably HES is provided therein as an aqueous solution.
39. Use of a hydroxyethyl starch according to any of embodiments 1 to 26 or a pharmaceutical composition according to embodiment 37 or 38 for the manufacture of a medicament, wherein HES is the only therapeutically active component for the treatment of cancer, which is characterized as reducing the growth rate of tumors.
40. Use of a hydroxyethyl starch according to embodiment 39, wherein the cancer is selected from the group according to embodiment 27, or from the group according to embodiment 28, or from the group according to embodiment 29, or according to embodiment 30, or according to embodiment 31, or according to embodiment 32, or from the group according to embodiment 33 or according to embodiment 34, or according to embodiment 35, or according to embodiment 36.
41. A method of treating a subject suffering from cancer comprising administering a therapeutically effective amount of a hydroxyethyl starch according to any of embodiments 1 to 26, or a pharmaceutical composition according to embodiment 37, thereby inhibiting progression of cancer, preferably by reducing the tumor growth rate, or more preferably by inhibiting the proliferation rate of tumor cells or by arresting the mitotic cell cycle of the tumor cell.
42. The method of embodiment 39 wherein the patient suffers from a cancer being selected from the group according to embodiment 27, or from the group according to embodiment 28, or from the group according to embodiment 29, or according to embodiment 30, or according to embodiment 31, or according to embodiment 32, or according to embodiment 33, 34, 35 or 36.
43. A method of preventing or treating cancer or metastatic disease in a subject, said method comprising administering an amount of the HES according to any one of embodiments 1 to 36 or a pharmaceutical composition according to embodiment 37 for a time and under conditions sufficient to ameliorate one or more adverse effects of cancer in a subject.
44. A hydroxyethyl starch according to any of the embodiments 1 to 26 for the treatment of cancers, wherein said cancers are characterized according to any of the embodiments 27 to 36, the treatment being characterized as being effective in reducing the tumor growth rate by reducing or inhibiting the proliferation rate of tumor cells.
45. A hydroxyethyl starch according to any of the embodiments 1 to 26 for the treatment of cancers, wherein said cancers are characterized according to any of the embodiments 27 to 36, the treatment being characterized as being effective in reducing the tumor growth rate by arresting the mitotic cell cycle of the tumor cells.

Description of figures:

Figure 1:
Figure 1 shows the body weight development of mice inoculated with MT-3 tumor cells over time as observed in Example 2. Values on the Y-axis indicate the body weight in gram, values on the X axis indicate the time in days after tumor cell inoculation. The substances are indicated by the following symbols: The small square is used when saline (NaCl) was administered to mice, indicated as Control. The large square is used when docetaxel, a known cytostatic, was administered to mice. The diamond symbol, is used when Voluven® 10% (1000mg/kg HES 105/0.4) was administered to mice, indicated as HES1. The triangle is used when HES 100/1.0 (approx. 735mg/kg HES 100/1.0) was administered to mice, indicated as HES2.

Figure 2:
Figure 2 shows the tumor volume development mice inoculated with MT-3 tumor cells over time as observed in Example 2. Values on the Y-axis indicate the body weight in gram, values on the X axis indicate the time in days after tumor cell inoculation. The substances are indicated by the following symbols: The small square is used when saline (NaCl) was administered to mice, indicated as Control. The large square is used when docetaxel, a known cytostatic, was administered to mice. The diamond symbol, is used when Voluven® 10% (1000mg/kg HES 105/0.4) was administered to mice, indicated as HES1. The triangle is used when HES 100/1.0 (approx. 735mg/kg HES 100/1.0) was administered to mice, indicated as HES2.

Figure 3:
Figure 3 shows the body weight development over time of the mice inoculated with MT-3 tumor cells in Example 1. Values on the Y-axis indicate the body weight in gram, values on the X axis indicate the time in days after tumor

cell inoculation. The substances are indicated by the following symbols: The small square is used when saline (NaCl) was administered to mice, indicated as Control. The large square is used when docetaxel, a known cytostatic, was administered to mice. The diamond symbol, is used when Voluven® 10% (1000mg/kg HES 105/0.4) was administered to mice, indicated as HES1. The triangle is used when HES 100/1.0 (approx. 735mg/kg HES 100/1.0) was administered to mice, indicated as HES2.

Figure 4:
Figure 4 shows the tumor volume development mice inoculated with MT-3 tumor cells over time as observed in Example 1. Values on the Y-axis indicate the body weight in gram, values on the X axis indicate the time in days after tumor cell inoculation. The substances are indicated by the following symbols: The small square is used when saline (NaCl) was administered to mice, indicated as Control. The large square is used when docetaxel, a known cytostatic, was administered to mice. The diamond symbol, is used when Voluven® 10% (1000mg/kg HES 105/0.4) was administered to mice, indicated as HES1. The triangle is used when HES 100/1.0 (approx. 735mg/kg HES 100/1.0) was administered to mice, indicated as HES2.

Figures 5 and 6 (Example 4):

Figure 5 shows the development of the relative tumor volume of mice inoculated with PC-3 tumor cells. Values on the Y-axis indicate the median relative tumor volume in per cent, values on the X-axis indicate the time in days after the start of treatment.
Figure 6 shows the development of the body weight of mice inoculated with PC-3 tumor cells. Values on the Y-axis indicate the mean body weight in gram ± the standard deviation (SD). Values on the X-axis indicate the time in days after the start of treatment.

For both figures, 5 and 6, the substances tested are indicated by the following symbols: the "▲" (black up-pointing triangle) is used when 0.9 % isotonic saline (NaCl) was administered to mice, indicated as "Control". The "■" (big black square) is used when Paclitaxel was administered to mice, indicated as "Taxol". The "♦" (black diamond) is used when "Voluven 10 % (2000 mg/kg HES 130/0.4)" was administered to mice, indicated as "HES".

Figures 7 and 8 (Example 5):

Figure 7 shows the development of the relative tumor volume of mice inoculated with MT-3 tumor cells. Values on the Y-axis indicate the median relative tumor volume in per cent. Values on the X-axis indicate the time in days after the start of treatment. The tumor volume was calculated after the exclusion of one mouse from HES 4 ("HES 100/1.0") group which was sacrificed at day 19 due to ethical reasons.
Figure 8 shows the development of the body weight of mice inoculated with MT-3 tumor cells. Values on the Y-axis indicate the mean body weight in gram ± the standard deviation (SD). Values on the X-axis indicate the time in days after the start of treatment.

In both figures, 7 and 8, the substances tested are indicated by the following symbols: the "▲" (black up-pointing triangle) is used when 0.9 % isotonic saline (NaCl) was administered to mice, indicated as "Control". The "■" (big black square) is used when Docetaxel (Taxotere®) was administered to mice, indicated as "Docetaxel". The "♦" (black diamond) is used when "HES 130/0.4" was administered to mice, indicated as "HES 1". The "◊" (white diamond) is used when HES 450/0.7 was administered to mice, indicated as "HES 2". The "●" (black circle) is used when HES 70/0.6 was administered to mice, indicated as "HES 3". The "○" (white circle) is used when "HES 100/1.0" was administered to mice, indicated as "HES 4".

Figures 9 and 10 (Example 5):

Figure 9 shows the development of the relative tumor volume of mice inoculated with MT-3 tumor cells. Values on the Y-axis indicate the median relative tumor volume in per cent. Values on the X-axis indicate the time in days after the start of treatment.
Figure 10 shows the development of the body weight of mice inoculated with MT-3 tumor cells. Values on the Y-axis indicate the mean body weight in gram ± the standard deviation (SD). Values on the X-axis indicate the time in days after the start of treatment.

In both figures, 9 and 10, the substances tested are indicated by the following symbols: the "▲" (black up-pointing triangle) is used when 0.9 % isotonic saline (NaCl) was administered to mice, indicated as "Control". The "■" (big black square) is used when Docetaxel (Taxotere®) was administered to mice, indicated as "Docetaxel". The "♦" (black diamond) is used when HES 450/0.7 was administered to mice, indicated as "HES 1". The "◊" (white diamond) is used when HES 700/0.7 was administered to mice, indicated as "HES 2". The "●" (black circle) is used when HES 700/0.5 was administered to mice, indicated as "HES 3". The "○" (white circle) is used when HES 730/1.3 was administered to mice, indicated as "HES 4". The "*" (black asterisk) is used when HES 200/0.5 was administered to mice, indicated as "HES 5".

Figures 11 and 12 (Example 5):

Figure 11 shows the development of the relative tumor volume of mice inoculated with MT-3 tumor cells. Values on the Y-axis indicate the median relative tumor volume in per cent. Values on the X-axis indicate the time in days after the start of treatment.
Figure 12 shows the development of the body weight of mice inoculated with MT-3 tumor cells. Values on the Y-axis indicate the mean body weight in gram ± the standard deviation (SD). Values on the X-axis indicate the time in days after the start of treatment.

In both figures, 11 and 12, the substances tested are indicated by the following symbols: the "▲" (black up-pointing triangle) is used when 0.9 % isotonic saline (NaCl) was administered to mice, indicated as "Control". The "■" (big black square) is used when Docetaxel (Taxotere®) was administered to mice, indicated as "Docetaxel". The "♦" (black diamond) is used when HES 100/0.7 was administered to mice, indicated as "HES 1". The "◊" (white diamond) is used when "Voluven 10 % (HES 130/0.4)" was administered to mice, indicated as "HES 2". The "●" (black circle) is used when HES 900/0.7 was administered to mice, indicated as "HES 3". The "○" (white circle) is used when HES 1300/0.7 was administered to mice, indicated as "HES 4". The "*" (black asterisk) is used when HES 900/0.4 was administered to mice, indicated as "HES 5".

Figures 13 and 14 (Example 6.1):

Figure 13 shows the development of the relative tumor volume of mice inoculated with LXFL 529 tumor cells. Values on the Y-axis indicate the median relative tumor volume in per cent. Values on the X-axis indicate the time in days after the start of treatment.
Figure 14 shows the development of the relative body weight of mice inoculated with LXFL 529 tumor cells. Values on the Y-axis indicate the median relative body weight in per cent. Values on the X-axis indicate the time in days after the start of treatment.

In both figures, 13 and 14, the substances tested are indicated by the following symbols: the "▲" (black up-pointing triangle) is used when 0.9 % isotonic saline (NaCl) was administered to mice, indicated as "Control". The "■" (big black square) is used when Everolimus was administered to mice. The "♦" (black diamond) is used when "Voluven® 10% (2000 mg/kg HES 130/0.4)" was administered to mice, indicated as "HES".

Figures 15 and 16 (Example 6.2):

Figure 15 shows the development of the relative tumor volume of mice inoculated with LXFE 397 tumor cells. Values on the Y-axis indicate the median relative tumor volume in per cent. Values on the X-axis indicate the time in days after the start of treatment.
Figure 16 shows the development of the relative body weight of mice inoculated with LXFE 397 tumor cells. Values on the Y-axis indicate the median relative body weight in per cent. Values on the X-axis indicate the time in days after the start of treatment. Values for day 18 of the groups "Control" and "Capecitabine" have been excluded because only 2 of 5 mice have been alive.

In both figures the tested substances are indicated by the following symbols: the "▲" (black up-pointing triangle) is used when 0.9 % isotonic saline (NaCl) was administered to mice, indicated as "Control". The "■" (big black square) is used when Capecitabine was administered to mice. The "♦" (black diamond) is used when Voluven® 10 % (2000

mg/kg HES 130/0.4) was administered to mice, indicated as "HES". The "◊" (white diamond) is used when a combination of Voluven® 10 % (2000 mg/kg HES 130/0.4) and Capecitabine was administered to mice, indicated as "HES/ Capecitabine".

Figures 17 and 18 (Example 6.3)

Figure 17 shows the development of the relative tumor volume of mice inoculated with RXF 2178 tumor cells. Values on the Y-axis indicate the median relative tumor volume in per cent. Values on the X-axis indicate the time in days after the start of treatment.
Figure 18 shows the development of the relative body weight of mice inoculated with RXF 2178 tumor cells. Values on the Y-axis indicate the median relative body weight in per cent. Values on the X-axis indicate the time in days after the start of treatment.

In both figures the tested substances are indicated by the following symbols: the "▲" (black up-pointing triangle) is used when 0.9 % isotonic saline (NaCl) was administered to mice, indicated as "Control". The "♦" (black diamond) is used when HES 450/0.7 was administered to mice, indicated as "HES".

**Example 1:**

Summary: Mice were either treated with a single i.v. injection of docetaxel (Taxotere®) at a dose of 25 mg/kg or with the plasma expander Voluven® (20ml/kg) or with "HES 100/1.0" (approx. 740mg/kg) dissolved in saline to determine tumor growth and body weight over the course of the experiment.

Substances:

Docetaxel (available under the name Taxotere®) was obtained from Sanofi-Aventis Deutschland GmbH, (Lot CHB D9C895; Berlin, Germany) and was stored in the dark at - 20°C until use. Voluven® (10% hydroxyethyl starch 105/0.4 in 0.9% sodium chloride for injection) was obtained as ready-to-use product from Fresenius Kabi Deutschland GmbH and was stored at room temperature until use. "HES 100/1.0" (as specified in table 2 above) was dissolved in saline.
The final solution of docetaxel was prepared immediately before injection by mixing an appropriate volume of the original stock solution provided by Sanofi (20mg/ml) with saline (0.9% NaCl, B. Braun Melsungen AG, Melsungen, Germany) as indicated in table 4 below. Saline was mixed with absolute ethanol to obtain an approx. 6.25% solution, the original vehicle of Taxotere®. Voluven® was used in the original formulation described above. All solutions were prepared and injected under sterile conditions.

Table 4:

| **Preparation of injection solutions** | | | | | | |
|---|---|---|---|---|---|---|
| Substance | Dose | Dose/Mouse | Stock | Saline(ml) | EtOH (ml) | Volume total (ml) |
| Saline/EtOH | 10ml/kg | ~250 µl | | 4.594 | 0.406 | 5.000 |
| Docetaxel (Taxotere®) | 25mg/kg | ~0.5 mg | 0.625 ml | 4.375 | | 5.000 |
| Voluven® 10% | 20ml/kg | ~500 µl | 10.000 ml | | | 10.000 |
| HES 100/1.0 | 740mg/kg | ~14.8 mg | 407.1 mg | 5.500 | | 5.500 |

Mice

Adult female NMRI:nu/nu mice (TACONIC Europe, Lille Skensved, Denmark) bred in the own (EPO) colony were used throughout the study. At the start of experiment they were 6-8 weeks of age and had an average body weight of approx. 25 to 30 g.
All mice were maintained under controlled and standardized barrier conditions. They were housed - maximum five mice/cage - in individually ventilated cages (Macrolon Typ-II, system Techniplast, Italy). The mice were held under following environmental conditions: 22±1°C room temperature, 50±10% relative humidity and 12 hour-light-dark-rhythm. They received autoclaved food and bedding (Ssniff, Soest, Germany) and acidified (pH 4.0) drinking water ad libitum.
Animals were randomly assigned to groups. At treatment initiation the animals received ear marks and each

cage was labelled with the cage number, study number and animal number per cage.

| Summary of animal conditions | |
|---|---|
| **Subject** | **Conditions** |
| Animals, gender and strain | female NMRI:nu/nu mice |
| Age | 6-8 weeks |
| Body weight | Approx. 25 to 30 g at the start of treatment |
| Supplier | EPO |
| Environmental Conditions | Strictly controlled and standardised barrier conditions, IVC System Techniplast DCC (TECNIPLAST DEUTSCHLAND GMBH) |
| Caging | Macrolon Type-II wire-mesh bottom |
| Feed type | Ssniff NM, Soest, Germany |
| Drinking water | Autoclaved tap water in water bottles (acidified to ph 4 with HCl) |
| Feeding and drinking time | Ad libitum 24 hours per day |
| Room temperature | 22±1°C |
| Relative humidity | 50±10% |
| Light period | Artificial; 12-hours dark/12 hours light rhythm (light 06.00 to 18.00 hours) |
| Health control | The health of the mice is examined at the start of the experiment and twice per day during the experiment |
| Identification | Ear mark and cage labels |
| Tumor model | MT-3, human breast cancer cells |

Tumor model

The human breast cell carcinoma line MT-3 is widely used for evaluating new anticancer drugs and novel therapeutic strategies. It was therefore selected for this study. MT-3 xenografts are growing relatively fast and uniform.

The MT-3 cell line was used for subcutaneous (s.c.) xenotransplantation in immune deficient female NMRI:nu/nu mice. The MT-3 breast carcinoma cell line was obtained from the tumor bank of the National Cancer Institute of the former USSR, WONZ, Moscow (Naundorf H. Rewasowa E. Fichtner I et al. Characterization of two human mammary carcinomas, MT-1 and MT-3, suitable for in vivo testing of ether lipids and their derivatives. Breast Cancer Res Treat. 1992; 23:87-95).

The cells are cryo-preserved within the EPO tumor bank. Cells were thawed, expanded by in vitro culture and transplanted s.c. as cell suspension ($5x10^6$ tumor cells/mouse in 200$\mu$l).

Study Design

On study day 0 each $5x10^6$ tumor cells were transplanted s.c. into the flank of each mouse. The animals were monitored for tumor growth and when tumors were palpable, generally between day 7 and day 10 after transplantation, mice were randomised into the treatment groups.

Table 5

| Treatment groups and results | | | | | | | | |
|---|---|---|---|---|---|---|---|---|
| **Group** | **Mice [n]** | **Substances** | Dose | Route/ day of dosing | Tumor volume (mean) at day 34 | Tumor volume (mean) nadir | Platelets x$10^6$ at day 10 | WBC x$10^6$ at day 10 |
| 1 | 14 | Saline + EtOH | 10ml/lg | i.v./7 | 1.745±1.0 95 | n.a. | 1385±151 | 6.66±2.87 |
| 2 | 14 | Docetaxel (Taxotere®) | 25mg/kg | i.v./7 | 0.930±0.3 95 | 0.072±0.047 | 1418±85 | 3.09±0.76 |
| 3 | 14 | Voluven® 10% | 20ml/kg | i.v./7 | 1.175±0.5 25 | 0.076±0.026 | 1259±154 | 6.22±1.57 |
| 4 | 14 | HES 100/1.0 | 740mg/kg | i.v./7 | 1.249±0.7 48 | 0.076±0.020 | 1098±112 | 6.52±2.50 |

The application volume was 10ml/kg (approx. 200μl/20g mouse) mouse body weight for vehicle, HES 100/1.0 and docetaxel and 20ml/kg for Voluven®. The route of administration was by intravenous (i.v.) injection into the tail vein.

Individual tumor diameters were measured two times weekly with a calliper. Tumor volumes were calculated according to V = (length x $(width)^2$)/2. For calculation of the relative tumor volume (RTV) the tumor volumes at each measurement day were related to the day of first treatment. At each measurement day the median and mean tumor volumes per group and also the treated to control (T/C RTV) values in per cent were calculated.

Individual body weights of mice were determined two times weekly and mean body weight per treatment group.

Blood samples were drawn from the retro-orbital sinus under iso-flurane anaesthesia three days after the treatment. Blood samples of 50 - 100μl were taken into tubes prepared with EDTA for haematological parameters from a subset of each group (generally 30-50%). The following parameters were determined: total leucocyte count (WBC), erythrocyte count (RBC), haemoglobin (HGB), haematocrit (HCT), mean corpuscular volume (MCV), mean corpuscular haemoglobin (MCH), mean corpuscular haemoglobin concentration (MCHC) and platelet count (PLT) using a Coulter Counter instrument.

Mice were sacrificed when tumors reached a mean size above 1 $cm^3$ per group. On the day of necropsy mice were sacrificed by cervical dislocation and inspected for gross organ changes.

Statistical Evaluation

Statistical evaluation of was performed with the U-test of Mann and Whitney using the Windows program STATISTICA 6. A significance level of $p < 0.05$ was used.

Results

All tumors in the control group (group 1) showed progressive growths. The single i.v. treatment of MT-3 breast cancer-bearing mice with 25 mg/kg of docetaxel induced a significant inhibition of tumor growth. Administration of HES 100/1.0 and Voluven® resulted an intermediate inhibitory activity which reached statistical significance ($p<0.05$) in a number of time points compared to the untreated control group and thereby demonstrated an inhibitory potential of ethylated starches on tumor growth rate (see figure 4).

Body weight loss was observed after treatment with docetaxel. General toxicity reached the maximum about 1 week after treatment. Later, mice recovered from that effect. Treatment with Voluven® or HES 100/1.0 had no significant effect on body weight development and thus no obvious treatment related toxicity. Autopsy at the end of experiment revealed no visible gross organ changes (see figure 3).

A slight but significant leucopoenia was observed after treatment with docetaxel. Leucopenia was not observed for Voluven® and HES 100/1.0 treatment (see table 5).

HES 100/1.0 caused a slight reduction in the number of platelets as compared to that of the saline + EtOH treated group, but the observed differences are of no biological relevance, because they remained within the standard variability observed in untreated animals. Docetaxel and Voluven® had no significant effect on the platelet counts. Tumor free nude mice had $7.6\pm2.2 \times 10^6$/ml leucocytes and $1761\pm311 \times 10^6$/ml platelets. Reference values given are 5.0 - 13.7 x 106/ml and 600 - 1200 x $10^6$/ml for leucocytes and platelets, respectively.

**Example 2**

Summary: Mice were either treated with a single i.v. injection of docetaxel (Taxotere®) at a dose of 25 mg/kg or with the plasma expander Voluven® (20ml/kg) or with HES 100/1.0 (approx. 740mg/kg) dissolved in saline/EtOH to determine tumor growth and body weight over the course of the experiment.

Substances:

Docetaxel (available under the name Taxotere®) was obtained from Sanofi-Aventis Deutschland GmbH, (Lot CHB D9C895; Berlin, Germany) and was stored in the dark at - 20°C until use. Voluven® (10% hydroxyethyl starch 105/0.4 in 0.9% sodium chloride for injection) was obtained as ready-to-use product from Fresenius Kabi Deutschland GmbH and was stored at room temperature until use. HES 100/1.0 (as specified in table 2 above) was dissolved in saline/EtOH.

The final solution of docetaxel was prepared immediately before injection by mixing an appropriate volume of the original stock solution provided by Sanofi (20mg/ml) with saline (0.9% NaCl, B. Braun Melsungen AG, Melsungen, Germany) as indicated in the table 6 below. Saline was mixed with absolute ethanol to obtain an approx. 6.25% solution, the original vehicle of Taxotere®. Voluven® was used in the original formulation described above. All solutions were prepared and injected under sterile conditions.

Table 6:

| Preparation of injection solutions | | | | | | |
|---|---|---|---|---|---|---|
| Substance | Dose | Dose/Mouse | Stock | Saline(ml) | EtOH (ml) | Volume total (ml) |
| Saline/EtOH | 10ml/kg | ~250 µl | | 3.975 | 0.525 | 4.500 |
| Docetaxel (Taxotere®) | 25mg/kg | ~0.5 mg | 0.525 ml | 3.675 | | 4.200 |
| Voluven® 10% | 20ml/kg | ~500 µl | 8.400 ml | | | 8.400 |
| HES 100/1.0 | 735mg/kg | ~14.7 mg | 331.2 mg | 3.975 | 0.525 | 4.500 |

Mice

Adult female NMRI:nu/nu mice (TACONIC Europe, Lille Skensved, Denmark) bred in the own (EPO) colony were used throughout the study. At the start of experiment they were 6-8 weeks of age and had an average body weight of approx. 25 to 30 g.

All mice were maintained under controlled and standardized barrier conditions. They were housed - maximum five mice/cage - in individually ventilated cages (Macrolon Typ-II, system Techniplast, Italy). The mice were held under following environmental conditions: 22±1°C room temperature, 50±10% relative humidity and 12 hour-light-dark-rhythm. They received autoclaved food and bedding (Ssniff, Soest, Germany) and acidified (pH 4.0) drinking water ad libitum.

Animals were randomly assigned to groups. At treatment initiation the animals received ear marks and each cage was labelled with the cage number, study number and animal number per cage. The animal conditions can be described as in Example 1.

Tumor Model

The human breast cell carcinoma line MT-3 is widely used for evaluating new anticancer drugs and novel therapeutic strategies. It was therefore selected for this study. MT-3 xenografts are growing relatively fast and uniform.

The MT-3 cell line was used for subcutaneous (s.c.) xenotransplantation in immune deficient female NMRI:nu/nu mice. The MT-3 breast carcinoma cell line was obtained from the tumor bank of the National Cancer Institute of the former USSR, WONZ, Moscow (Naundorf H. Rewasowa E. Fichtner I et al. Characterization of two human mammary carcinomas, MT-1 and MT-3, suitable for in vivo testing of ether lipids and their derivatives. Breast Cancer Res Treat. 1992; 23:87-95).

The cells are cryo-preserved within the EPO tumor bank. Cells were thawed, expanded by in vitro culture and transplanted s.c. as cell suspension ($5x10^6$ tumor cells/mouse in 200µl).

Study Design

On study day 0 each $5x10^6$ tumor cells were transplanted s.c. into the flank of each mouse. The animals were monitored for tumor growth and when tumors were palpable, about day 7 after inoculation, mice were randomised into the treatment groups.

Table 7

| Group | Mice [n] | Substances | Dose | Route | Day of dosing after inocualtion | Tumor volume (mean) $cm^3$/day 25 | Platelets $x10^6$ at day 11 | WBC $x10^6$ at day 11 |
|---|---|---|---|---|---|---|---|---|
| 1 | 8 | Saline + EtOH | 10ml/kg | i.v. | 8 | 1.16 | 1367±175 | 3.75±1.53 |
| 2 | 8 | Docetaxel (Taxotere®) | 25mg/kg | i.v. | 8 | 0.343 | 1500±156 | 4.80±0.58 |
| 3 | 8 | Voluven® 10% | 20ml/kg | i.v. | 8 | 0.854 | 1442±175 | 9.30±4.54 |
| 4 | 8 | HES 100/1.0 | 735mg/kg | i.v. | 8 | 0.788 | 1382±102 | 5.03±1.02 |

The application volume was 10ml/kg (approx. 200μl/20g mouse) mouse body weight for vehicle, HES 100/1.0 and docetaxel and 20ml/kg for Voluven®. The route of administration was by intravenous (i.v.) injection into the tail vein.
Individual tumor diameters were measured two times weekly with a calliper. Tumor volumes were calculated according to V = (length x (width)$^2$)/2. For calculation of the relative tumor volume (RTV) the tumor volumes at each measurement day were related to the day of first treatment. At each measurement day the median and mean tumor volumes per group and also the treated to control (T/C RTV) values in per cent were calculated.
Individual body weights of mice were determined two times weekly and mean body weight per treatment group.

Blood Parameters

Blood samples were drawn from the retro-orbital sinus under iso-flurane anaesthesia three days after the treatment. Blood samples of 50 - 100μl were taken into tubes prepared with EDTA for haematological parameters from a subset of each group (generally 30-50%). The following parameters were determined: total leucocyte count (WBC), erythrocyte count (RBC), haemoglobin (HGB), haematocrit (HCT), mean corpuscular volume (MCV), mean corpuscular haemoglobin (MCH), mean corpuscular haemoglobin concentration (MCHC) and platelet count (PLT) using a Coulter Counter instrument.

Clinical chemistry parameters

Blood samples of 150 - 200 μl were collected without EDTA from a further subset (generally 30-50%) of the groups and serum generated by centrifugation. Serum samples were analysed and the following parameters determined: Alkaline phosphatase (AP), glutamic oxalacetate transaminase (GOT), glutamate pyruvat transaminase (GPT), glutamate dehydrogenase (GLDH), bilirubin total (BIL), lactate dehydrogenase (LDH), creatinine kinase (CK), cholesterol (CHOL), triglyceride (TG), creatinine (CREA), urea, sodium, potassium, magnesium (Mg), phosphate, calcium/phosphate quotient (Ca/P-quotient), glucose, albumin, albumin/globulin quotient (Alb/Glob-quotient) and total protein (TP).
Mice were sacrificed when tumors reached a mean size above 1 cm$^3$ per group. On the day of necropsy mice were sacrificed by cervical dislocation and inspected for gross organ changes.

Statistical Evaluation

Statistical evaluation of was performed with the U-test of Mann and Whitney using the Windows program STATISTICA 6. A significance level of $p < 0.05$ was used.

Results

Table 8

| | tumor size at day 8 [cm$^3$] | tumor size at day 10 [cm$^3$] | tumor size at day 15 [cm$^3$] | tumor size at day 18 [cm$^3$] | tumor size at day | tumor size at day |
|---|---|---|---|---|---|---|
| Control | 0,0865 | 0,1785 | 0,4765 | 0,7120 | 1,0191 | 1,1600 |
| Docetaxel | 0,0866 | 0,1046 | 0,1228 | 0,1995 | 0,2465 | 0,3430 |
| Voluven | 0,0852 | 0,1450 | 0,3806 | 0,4574 | 0,7093 | 0,8545 |
| HES 100/1.0 | 0,0875 | 0,1246 | 0,4270 | 0,5043 | 0,7460 | 0,7886 |

All tumors in the control group (group 1) showed progressive growths. The single i.v. treatment of MT-3 breast cancer-bearing mice with 25 mg/kg of docetaxel induced a significant inhibition of tumor growth. Administration of HES 100/1.0 and Voluven® resulted an intermediate inhibitory activity which reached statistical significance ($p<0.05$) in a number of time points compared to the untreated control group and thereby demonstrated an inhibitory potential of ethylated starches on tumor growth rate (see table 8 and figure 2).
Body weight loss was observed after treatment with docetaxel. General toxicity reached the maximum about 1 week after treatment. Later, mice recovered from that effect. Treatment with Voluven® or HES 100/1.0 had no significant

effect on body weight development and thus no obvious treatment related toxicity (see figure 1).

Autopsy at the end of experiment revealed no visible gross organ changes.

Clinical chemistry analysis revealed a change in different parameters, but no clear correlation to the treatment can be detected.

Example 3 (hypothetical) deleted

**Example 4:**

In another study which was performed in analogy to the study described above, the effect of "Voluven 10%" was tested on a different cancer cell line, i.e. on prostate cancer cell line PC3.

The rapidly growing human prostate carcinoma cell line PC-3 was used as xenograft tumor model in nude mice to test the efficacy of Voluven® 10% on reduction of tumour growth rates. Paclitaxel was employed as positive control and saline as negative control. The results showed that Voluven® 10% was effective in the PC-3 model as indicated by a 2-fold lower relative tumour volume compared to saline. The positive control paclitaxel showed stable tumour size or reduced the tumor volume within the 3 week observation period.

Substances

Voluven® 10%:

Supplier: Fresenius Kabi Deutschland GmbH
Batch number: 14EC3320
Expiration date: March 2014
Physical state/color: liquid, colorless
Storage conditions: 15 to 25 °C

Paclitaxel

Supplier: Aurigon Life Science GmbH
Manufacturer: Oncotrade
Batch number: V407 + 12634403
Expiration date: February 2013 + May 2013
Physical state/color: Liquid, colorless to slightly yellow
Storage conditions: 15 to 25 °C

Vehicle

Name: 0.9 % isotonic saline
Supplier: B.Braun Melsungen AG
Physical state/color: Liquid, colorless
Storage conditions: 15 to 25 °C

Mice

Strain: NMRI nude (homozygous) -Rj :NMRI-nu
Sex: Female
Supplier: Elevage Janvier, Route des Chènes Secs, 53940 Le Genest St. Isle, France
Health status: SPF
Age at delivery: 5-6 weeks

Acclimatization: At least 6 days

The mouse is a suitable rodent species for anti-tumor research and is accepted by regulatory authorities. As no gender-specific differences were expected only female animals were used in this study. The intravenous administration route was chosen as it corresponds to the route of administration in humans.

The animals were housed in individually ventilated (IVC) Makrolon® cages of type II with five at most animals per cage. The room temperature was adjusted to 22 ± 3 °C and the relative humidity to 50% ± 20%. These parameters were recorded daily. Artificial light was set to give a light/dark cycle of 12 hours (LD 12:12) with light on at 6:30 a.m. Air was changed about 10 times per hour in the animal room and about 70 times per hour in the individually ventilated cages filtered adequately. The animals received gamma radiated pelleted diet (ssniff R/M-H) produced by ssniff Spezialdiäten GmbH (Experimental Animal Diets Inc. 59494 Soest, Germany) ad libitum. Drinking water sterilized by autoclaving was continuously available ad libitum via drinking bottles. The bedding used (Lignocel® type FS14) was supplied by ssniff Spezialdiäten GmbH (Soest, Germany) and autoclaved before use. It was renewed usually once a week.

Tumor model

Name: PC-3
Tissue: Human prostate carcinoma
Cell type: Adherent
Supplied by: DSMZ
Culture medium: DMEM/HAM's F12 (1:1) supplemented with 10% fetal bovine serum
Culture conditions: 37°C, 5% CO2
Splitting factor: 1:3 to 1:10

Study design

In total 45 animals per tumor model were inoculated with the PC-3 cell suspensions. On the day of inoculation cells were washed once or twice with culture medium (RPMI 1640 without additives) and harvested by standard procedures. Final cell concentrations were adjusted on the day of inoculation to $5x10^6$ cells in 70 μl PBS per animal for PC-3 cells for subcutaneous injection into the right flank. Animals with the smallest and largest tumors were excluded and taken out of the study at the day of group formation (3x 10 animals per tumor model). The groups were built with comparable mean tumor sizes within the model (PC-3: 80-100 $mm^3$) and standard deviations for the tumor size.

Tumor measurement: The tumor growth was measured three times a week (e.g. Monday, Wednesday, and Friday) beginning with study day 3 (tumor measurable at size approx. 3 mm in diameter). Tumor volumes were calculated according to V = (length x $(width)^2$)/2. For calculation of the relative tumor volume (RTV) the tumour volumes at each measurement day were related to the day of first treatment.

To prepare the paclitaxel solution at a concentration of 2.5 mg/ml the paclitaxel stock solution (6 mg/ml) was diluted with 0.9 % isotonic saline. The formulation was generated freshly on each day of treatment. Any unused formulation was discarded after the application. Saline and Voluven® 10% were provided as ready-to-use formulations.

Animals were treated intravenously by a slow bolus administration into the lateral tail vein once weekly for three weeks with saline or paclitaxel (25 mg/kg) or daily with Voluven® 10% over 21 days starting with study day 0. Dosing volume was 10ml/kg.

Mice were sacrificed after 21 days and a macroscopic necropsy performed (inspection for gross organ changes).

Statistical evaluation

Statistical analyses of data were performed for each group separately. Group medians were calculated for body weights and tumor measurements for each investigation time. Test item treated groups were compared to the reference groups and vehicle groups for the PC-3 cell line independently. Body weights and tumor measurements were evaluated using ToxData® System (version 3) with the statistical calculation of the device (decision tree). As a level of significance, $p<0.05$ was accepted. For the evaluation of tumor volume/area and T/C values were calculated using Microsoft Excel 7.0.

Results

The rapidly growing human prostate carcinoma cell line PC-3 was sensitive to the reference paclitaxel as indicated by a decrease of the initial tumor volume over the 3 weeks observation period. The saline negative control

group showed an approx. 5-fold increase in relative tumor volume within this period. Administration of the test item "Voluven® 10%" once daily over 3 weeks resulted in clearly decreased tumor growths compared to the saline group with an approx. 3-fold initial tumor volume. Body weight development was about stable with slight increase over the observation period.

The result is illustrated in Figures 5 (tumor volume) and 6 (body weight).

**Example 5:**

In another set of three studies which were performed in analogy to the studies described above in Examples 1 and 2, more different HES types have been analysed in the same setting using the MT3 cell line mouse model.

The HES types tested in three different studies in the same model were the following:

Table 11:

| Substance "Name" Mw/Ms/PDI | Figure | Dose | Dose/mouse (20 g) | Day of dosing |
|---|---|---|---|---|
| Saline<br>- | Neg Control in all figures | 20 ml/kg | 400 μl | 0,7,14 |
| Docetaxel (Taxotere®)<br>- | Pos Control in all figures | 40 mg/kg | 800 μg | 0,14 |
| "HES 70/0.5"<br>66/0.57/2.3 | Fig 7, Fig 8 | 20 ml/kg | 400 μl | 0,7,14 |
| "HES 100/0.7/1.3"<br>103/0.7/1.3 | Fig 11, Fig 12 | 20 ml/kg | 400 μl | 0,7,14 |
| "HES 100/1.0/1.3"<br>78/1.0/1.4 | Fig 7, Fig 8 | 20 ml/kg | 400 μl | 0,7,14 |
| "HES 130/0.4"<br>105/0.4 (dissolved in saline) | Fig 7, Fig 8 | 20 ml/kg | 400 μl | 0,7,14 |
| "Voluven 10% 130/0.4"<br>105/0.4 | Fig 11, Fig 12 | 20 ml/kg | 400 μl | 0,7,14 |
| "HES 200/0.5"<br>195/0.46 | Fig 9, Fig 10 | 20 ml/kg | 400 μl | 0,7,14 |
| "HES 450/0.7"<br>420/0.7 | Fig 7, Fig 8 | 20 ml/kg | 400 μl | 0,7,14 |
| "HES 700/0.5"<br>618/0.5/2.2 | Fig 9, Fig 10 | 20 ml/kg | 400 μl | 0,7,14 |
| "HES 700/0.7"<br>644/0.7/1.9 | Fig 9, Fig 10 | 20 ml/kg | 400 μl | 0,7,14 |
| "HES 700/1.3"<br>728/1.3/1.6 | Fig 9, Fig 10 | 20 ml/kg | 400 μl | 0,7,14 |
| "HES 900/0.4"<br>929/0.4/3.0 | Fig 11, Fig 12 | 20 ml/kg | 400 μl | 0,7,14 |
| "HES 900/0.7"<br>1034/0.67/3.0 | Fig 11, Fig 12 | 20 ml/kg | 400 μl | 0,7,14 |
| "HES 1300/0.7"<br>1406/0.7/4.4 | Fig 11, Fig 12 | 20 ml/kg | 400 μl | 0,7,14 |

The HES type with the name HES 100/1.0/1.3 is characterized in more detail in table 3.

It is hereby shown that a wide range of different HES types effectively reduces the growth rates of tumors in vivo, compared to the growth rate when administering saline as control. The results are disclosed in Figures 7, 9 and 11 (tumor volume) and 8, 10 and 12 (body weight).

Substances

Docetaxel (Taxotere®) was obtained from Sanofi-Aventis Deutschland GmbH, (Lot CHB D9C895; Berlin, Germany) and was stored in the dark at -20 °C until use. The injection solution was prepared immediately before use by dissolving in saline solution. Hydroxyethyl starch compounds were obtained from Fresenius Kabi Deutschland GmbH on 12/07/2012 and were stored at room temperature until use. The compounds each were dissolved in saline to obtain 10 % w/v solutions. The application was 400 μl/20 g mouse body weight for saline and HES solutions, while Docetaxel was given in a volume of 10 ml/kg as slow bolus. The route of administration was intravenously for all mice.

Mice

Adult female NMRI:nu/nu mice (TACONIC Europe, Lille Skensved, Denmark) bred in the own (EPO) colony were used throughout the study. At the start of experiment they were 6-8 weeks of age.

All mice were maintained under controlled and standardized barrier conditions. They were housed - maximum five mice/cage - in individually ventilated cages (Macrolon Typ-II, system Techniplast, Italy). The mice were held under following environmental conditions: 22 ± 1 °C room temperature, 50 ± 10 % relative humidity and 12 hour-light-dark-rhythm. They received autoclaved food and bedding (Ssniff, Soest, Germany) and acidified (pH 4.0) drinking water ad libitum.

At treatment initiation the animals received ear marks and each cage was labelled with the cage number, study number, and animal number per cage.

The animal conditions were the same as in Examples 1 and 2.

The mice for study 1 of this series had at this time a median body weight of 23.6 g (19.7 - 23.4g). The tested substances were HES 70/0.5, HES 100/1.0, HES 130/0.4 and HES 450/07.

The mice for study 2 of this series had at this time a median body weight of 27.1g (22.1 - 33.2g). The tested substances in study 2 were HES 700/1.3, HES 700/0.5/2.5, HES 700/0.7, HES 450/0.7 and HES 200/0.5.

The mice for study 3 of this series had at this time a median body weight of 25.04 g (16.9 to 30.4 g). The tested substances were HES 100/0.7/1.3, HES 900/0.4, HES 900/0.7, HES 1300/0.7 and Voluven 10%.

Tumor Model

The human breast cell carcinoma line MT-3 is widely used for evaluating new anticancer drugs and novel therapeutic strategies. It was therefore selected for this study. MT-3 is a fast growing tumour model and develops palpable tumour nodules after approx. 7 days. The MT-3 cell line was used for subcutaneous (s.c.) xenotransplantation in immune deficient female NMRI:nu/nu mice. The MT-3 breast carcinoma cell line was obtained from the tumour bank of the National Cancer Institute of the former USSR, WONZ, Moscow.

The cells are cryo-preserved. Cells were thawed, expanded by in vitro culture and transplanted s.c. as cell suspension in female NMRI:nu/nu mice.

Study design

The number of $5x10^6$ tumour cells was transplanted s.c into the flank of each mouse. The animals were monitored for tumour growth and when tumours were palpable, generally between day 7 and day 10 after transplantation, mice were randomised into the treatment groups with 15 mice each. Day 0 is defined as the day of first treatment.

Tumor measurement

Individual tumour diameters were measured twice weekly with a calliper-like instrument. Tumour volumes were calculated according to $V = (\text{length} \times (\text{width})^2)/2$. At each measurement day the median relative tumour volumes per group were determined and are displayed in the figures 7, 9 and 11. The relative tumour volume (RTV) and the treated to control (T/C RTV) values in per cent were calculated for each measurement day in relation to V values at day 0.

Body weight

Individual body weights of mice were determined twice weekly and mean body weight per treatment group were calculated and displayed in the figures 8, 10 and 12.

End of experiment

In studies 1 and 2 mice were sacrificed when tumours reaches more than 2 $cm^3$, the median tumour volume is increased by 3-5 fold, a consistent or rapid body weight loss of 20 % maintained for 72 hours is observed or the purpose of the experiment is reached. In study 3 mice were sacrificed when the tumor size reached more than 1.5 $cm^3$, or the tumors ulcereted. Study 3was finished when the mean TV was in most groups larger than 1.0 $cm^3$.
On the day of necropsy mice were sacrificed by cervical dislocation and inspected for gross organ changes.

Results

Mice were treated with i.v. injection of Docetaxel (Taxotere®) at day 0 and 14 at a dose of 40 mg/kg and with the hydroxyethyl starch compounds (20 ml/kg) once weekly.
In study 1 all mice developed tumours after s.c. transplantation of $5x10^6$ MT-3 cells up to a size of 0.046 ± 0.015 $cm^3$ within 7 days. Mice were treated intravenously with a dose of 40 mg/kg Docetaxel (Taxotere®) at days 0 and 14, whereas four different HES specifications in saline (containing each 10 % w/v) were injected in a volume of 20 ml/kg i.v. weekly (day 0, 7, 14). Saline was injected once a week (20 ml/kg). All groups were treated during the whole experimental period accordingly to the planned schedule. The tumour of control mice in study 1 grew up to a volume of 1.1 ± 0.6 $cm^3$.
In study 2 all mice developed tumours after s.c. transplantation of $5x10^6$ MT-3 cells up to a size of 0.081 ± 0.033 $cm^3$ within 9 days. Mice were treated intravenously with a dose of 40 mg/kg docetaxel at days 0 and 14, whereas four different HES specifications in saline (containing each 10 % w/v) were injected in a volume of 20 ml/kg i.v. weekly (day 0, 7, 14). Saline was injected once per week (20 ml/kg). All groups were treated during the whole experimental period accordingly to the planned schedule. The tumour of control mice grew up until day 18 to a volume of 1.48 ± 1.04 $cm^3$.
In study 3 all mice developed tumours after s.c. transplantation of 5x106 MT-3 cells up to a size of 0.059 ± 0.018 cm3 within 7 days. Mice were treated intravenously with a dose of 40 mg/kg docetaxel at days 0 and 14, whereas five different HES specifications (including Voluven®) in saline (containing each 10% w/v) were injected in a volume of 20 ml/kg i.v. weekly (d0, 7 and 14). Saline was injected once per week (20 ml/kg). All groups were treated during the whole experimental period accordingly to the planned schedule. The tumour of control mice in study 3 grew up until day 14 to a volume of 0.805±0.547 $cm^3$
Docetaxel was a well-chosen positive control. It had a strong inhibitory effect on tumour growth with an inhibition of more than 85 % (14.4 % RTV T/C) in study 1; 83% (16.9% RTV T/C) in study 2 and 91% (8.8% RTV T/C) at day 7 in study 3.
Surprisingly, treatment with all HES specifications tested also resulted in a tumour growth inhibition effect, as can be seen in the figures 7, 9 and 11. In study 1 especially significant effects could be observed for HES 450/0.7 with an RTV T/C values of 34.4 % and HES 100/1.0 with an RTV T/C value of 56.9 %. The most active compound was HES 450/0.7, which induced a significant inhibition from day 4 until the end of the experiment. In study 2 especially significant effects could be observed for HES 200/0.5 with an RTV T/C value of 60.5 % and HES 700/0.7 with an RTV TC value of 76.3 %. The most active compound was HES 200/0.5.
In study 3 especially s significant effects could be observed for HES 100/0.7/1.3 which resulted in a tumour growth inhibition effect with RTV T/C values between 55.8% and HES130/0.4, Voluven® 10% with a RTV T/C value of 89.1%. The most active compound was HES 100/0.7/1.3

General toxicity

There was no drug related toxicity observed during study 1. All treatments were well tolerated without body weight loss. Only a transient delayed body weight growth was observed at specific days during the experimental period.
In study 2 Docetaxel given i.v. at day 0 and day 14 caused apathy. After the first treatment with Docetaxel a strong body weight loss was observed at day 6.
In study 3 Docetaxel given i.v. caused a moderate body weight loss. The maximum of 6.5% was observed at day 7 after the first treatment.
All treatments with HES specifications were well tolerated without any body weight changes in all three studies. (Fig 8, 10 and 12).
Autopsy at the end of the experiment revealed no gross organ changes.

**Example 6:**

In another study it was analysed whether this effect could be observed at a number of different tumor types. This study was performed with the "Oncotest Model", that is explained below:

In these studies either the HES type commercially available under the name Voluven 10%, a "HES 130/04" as described in detail above was used, or a "HES 450/0.7", as described in detail in the specification with a Mw of 420 kDa measured according to European Pharmacopoeia, with a Dn/dc of 0.147 and a Ms of 0.7.

The patient-derived tumor models (xenografts) used by Oncotest were derived from surgical specimens from patients. The cells from this tumor tissue are not transferred into cell culture. Instead the tumor cells are passaged from the human patient to a mouse (passage 1) and then from mouse to mouse in form of the tumor tissue. This setting bears the advantage that it more closely resembles the real 'behaviour' of a tumor compared to a cell line which was amplified *in vitro* over many years.

Briefly, following their primary implantation into nude mice (passage 1, P1), the tumor xenografts were passaged (from mouse to the next mouse) until establishment of stable growth patterns. Stocks of early passage xenografts were frozen in liquid nitrogen. Usually, only passage numbers below 30, if available preferably below 20, were used for compound testing.

Animals and tumor implants were monitored daily until the maximum number of implants show clear signs of beginning solid tumor growth. At randomization of the animals, for example into different cages, the volume of growing tumors was initially determined. If not stated elsewhere, animals bearing at least one tumor of a volume of 50 - 250 $mm^3$, preferably 80 - 200 $mm^3$, were distributed in experimental groups according to the study protocol, considering a comparable median and mean of group tumor volume. The day of randomization was designated as day 0 of an experiment and was also the first day of dosing.

The tumor volume doubling time (DT), defined as the time interval (in days) required for a group of mice to reach a median RTV (relative tumor volume) of 200% of the initial tumor volume (normally from 100-200 mm3), is routinely recorded in untreated or vehicle treated control groups of experiments and a median DT is calculated for characterization purposes.

| Tumormodel | Median doubling time (DT) | Growth characteristic |
|---|---|---|
| LXFL 529 | 4-6 | intermediately fast growing |
| LXFE 397 | 1-4 | Fast growing |
| RXF 2178 | 1-4 | fast growing |

The tumor volume was determined by a two-dimensional measurement with callipers on the day of randomization (Day 0) and then once to twice weekly. Tumor volumes were calculated according to the following equation:

$$\text{Tumor Vol } [mm^3] = a\ [mm] \times b^2\ [mm^2] \times 0.5$$

where "a" is the largest diameter and "b" is the perpendicular diameter of the tumor representing an idealized ellipsoid.

The relative volume of an individual tumor on day X ($RTV_x$) was calculated by dividing the absolute volume [$mm^3$] of the respective tumor on day X ($T_x$) by the absolute volume of the same tumor on the day of randomization, i.e. on day 0 ($T_0$), multiplied by 100, as shown by the following equation:

$$RTV_x\ [\%] = \frac{T_x}{T_0} \times 100$$

Group median (alternatively geometric mean +/- SD) of RTVs were calculated, considering only the tumors of animals that were alive on the day in question (for median). Group median (geometric mean) RTVs are used for drawing tumor growth curves.

Starting on day 0, animals were weighed once to twice a week. Relative body weights (RBW) of individual animals are calculated by dividing the individual absolute body weight on day X ($BW_x$) by the individual body weight on the day of randomization, i.e. day 0 ($BW_0$), multiplied by 100, as shown by the following equation:

$$RBW_x\ [\%] = \frac{BW_x\ [g]}{BW_0\ [g]} \times 100$$

Group median (alternatively arithmetic mean +/- SD) of relative body weights were calculated, considering only the weights of animals that were alive on the day in question (for median).

The following termination criteria were applied to individual animals, irrespective of experimental status:

- tumor volume > 2000 $mm^3$ (unilateral)
- animals bearing ulcerating, skin-penetrating tumor
- body weight loss > 30%
- continued body weight loss > 20% for more than 7 days
- severe impairment of general condition (apathy, pain, markedly reduced feed and water intake, dyspnoea, abnormal habitus or behaviour)
- Whole groups were will be terminated if less than 3 animals were are left.

6.1 LXFL 529

Mice were either treated with Everolimus at a dose of 10 mg/kg per orally (p.o.) or with the plasma expander "Voluven 10%" at a dose of 20 ml/kg intravenously (i.v.) or with saline (20 ml/kg) i.v. at days 0, 3, 7, 10, 14, and 17. Tumor growth and body weight were determined over the course of the experiment.

Everolimus (Fresenius Kabi, lot: 1101012750e) was stored at ≤ -18 °C until use. Voluven® 10% (lot: 14EC3320) was obtained as ready-to-use product from Fresenius Kabi Deutschland GmbH and was stored at ambient temperature until use. Saline (0.9 % NaCl) was obtained from AlleMan Pharma.

The final solution of Everolimus was prepared by mixing equal amounts of mg compound and ml vehicle (N-methylpyrrolidone, Sigma Aldrich, lot: STBB6784 / PEG300, FLUKA, lot: BCBJ0244V; 1:9) immediately before injection. Saline and Voluven were used in the original formulation. All solutions were prepared and injected under sterile conditions.

Tumor implants of the human tumor xenograft, LXFL 529/32N24 (Non-Small Cell Lung Cancer, Large Cell), which were derived from a primary tumor in the lung, poorly differentiated, were implanted subcutaneously (s.c.) into the left flank of immunodeficient female NMRI nu/nu mice under isoflurane anaesthesia. This tumor xenograft has a recorded median doubling time of 4-6 days.

Table 12

| Treatment groups | | | | |
|---|---|---|---|---|
| Group | Mice [n] | Substances | Dose | Days of dosing |
| **1** | 5 | Saline | 20 ml/kg | 0,3,7,10,14,17 |
| **2** | 5 | Everolimus | 10 mg/kg | 0,3,7,10,14,17 |
| **3** | 5 | Voluven® 10% | 20 ml/kg | 0,3,7,10,14,17 |

The application volume was 20 ml/kg mouse body weight for saline and Voluven® 10% and 10 ml/kg of the 1 mg/ml solution for Everolimus (administered final dose 10 mg/kg). The route of administration was by i.v. injection into the tail vein for saline and Voluven® 10%. 10 mg/kg of body weight Everolimus was administered p.o..

Results

As illustrated in Figure 13 after treatment with Voluven the tumor size of the treated mice was reduced compared to the tumor size after the same time in mice treated with saline only. At the end of the treatment phase, at day 21, the tumor size was even smaller than in the treatment group that received Everolimus, the "standard of care" drug. In Figure 14 it is illustrated, that the body weight of the treated animals was not reduced due to treatment with Voluven, whereas a slight reduction occurred in animals which received Everolimus.

6.2. LXFE 397

Mice were either treated with Capecitabine at a dose of 100 mg/kg per orally (p.o.), with the plasma expander Voluven® 10% at a dose of 20 ml/kg intravenously (i.v.), or with saline (20 ml/kg) i.v. at days 0, 4, 7, 11, 14, and 18. Tumor growth and body weight were determined over the course of the experiment.

Capecitabine (Xeloda®, Roche, lot: X9062B01) was stored at ambient temperature until use. Voluven® 10% (lot: 14EC3320) was obtained as ready to use product from Fresenius Kabi Deutschland GmbH and was stored at ambient temperature until use. Saline (0.9 % NaCl) was obtained from AlleMan Pharma.

The final solution of Capecitabine was prepared by dissolving 60 mg of compound in 6 ml of aqua ad iniectabilia

(AlleMan Pharma) to obtain a solution with a concentration of 10 mg/ml immediately before injection. Saline and Voluven® were used in the original formulation. All solutions were prepared and injected under sterile conditions.

Tumor implants of the human tumor xenograft, LXFE 397/26N17 (Non-Small Cell Lung Cancer, histologically characterised as squamous cell carcinoma), derived from a primary tumor in the lung, poorly differentiated, were implanted subcutaneously (s.c.) into the left flank of immunodeficient female NMRI nu/nu mice under isoflurane anaesthesia. This tumor xenograft has a recorded doubling time of 1-4 days.

Table 13

| **Treatment groups** | | | | |
|---|---|---|---|---|
| **Group** | **Mice** [n] | **Substances** | **Dose** | **Days of dosing** |
| **1** | 5 | Saline | 20 ml/kg | 0,4,7,11,14,18 |
| **2** | 5 | Capecitabine | 100 mg/kg | 0,4,7,11,14,18 |
| **3** | 5 | Voluven® 10% | 20 ml/kg | 0,4,7,11,14,18 |

The application volume was 20 ml/kg mouse body weight for saline and Voluven® 10% (i.v.) and 100 mg/kg for Capecitabine (p.o.).

Results

After treatment with Voluven the tumor size of the treated mice was reduced compared to the tumor size after the same time in mice treated with saline only. At day 14, the tumor size was even smaller than in the treatment group that received Capecitabine, the "standard of care" drug, as illustrated in Figure 15. In Figure 16 it is illustrated, that the body weight of the treated animals was not reduced due to treatment with Voluven, nor when treated with Capecitabine.

6.3. RXF 2178

Mice were either treated with with a hydroxyethyl starch (HES 450/0.7) at a dose of 20 ml/kg of a 10 % solution i.v., or with saline (20 ml/kg) i.v. at days 0, 2, 4, 7, 9, 11, 14. HES 450/0.7, characterized in table 14 was obtained from Fresenius Kabi Austria (Linz) and was stored at ambient temperature until use. Saline (0.9 % NaCl) was obtained from AlleMan Pharma.

Table 14

| Test parameter | Measured properties of "HES 450/07" |
|---|---|
| Appearance | powder |
| Colour | white to yellowish white |
| Absorption 420 nm/1cm | 0.012 |
| Mw | 419.488 kDa |
| Mw of the 10% smallest fraction | 25.857 kDa |
| Mw of the 10% largest fraction | 1771.766 kDa |
| MS | 0.70 |

Tumor implants of the human tumor xenograft, RXF 2178 (renal cancer, histology: clear cell renal cell cancer; which were derived from a metastatic kidney tissue were implanted subcutaneously (s.c.) into the left flank of immunodeficient female NMRI nu/nu mice under isoflurane anaesthesia. This tumor xenograft has a median doubling time of 1-4 days.

Table 15:

| Treatment | | | | | |
|---|---|---|---|---|---|
| **Group** | **Mice** [n] | **Substances** | **Dose** | **Route** | **Days of dosing** |
| **1** | 5 | Saline | 20 ml/kg | i.v. | 0,2,4,7,9,11 |
| **2** | 5 | HES (450/0.7) | 20 ml/kg | i.v. | 0,2,4,7,9,11 |

The application volume was 20 ml/kg mouse body weight for saline, and 20 ml/kg mouse body weight for a 10% w/V solution of HES 450/0.7 (i.v.).

Tumor growth and body weight were determined over the course of the experiment.

Results

During the whole treatment course the tumor size of the mice treated with HES 450/0.7 was clearly reduced compared to the tumor size in mice treated with saline only, as illustrated in Figure 17. In Figure 18 it is illustrated, that the body weight of the treated animals was not significantly reduced due to treatment with HES 450/0.7.

## Claims

1. A pharmaceutical composition comprising a hydroxyethyl starch (HES), wherein HES is the only therapeutically active compound for use in the treatment of cancer, wherein the treatment is characterized as reducing tumor growth rates.

2. A pharmaceutical composition comprising a hydroxyethyl starch (HES) according to claim 1, for use in the treatment of cancer according to claim 1, wherein the cancer is selected from the group of solid tumors, which are tumors arising in solid tissues.

3. A pharmaceutical composition comprising a hydroxyethyl starch (HES) according to claims 1 or 2, for use in the treatment of cancer according to claim 1, wherein the cancer is selected from the group of carcinoma.

4. A pharmaceutical composition comprising a hydroxyethyl starch according to any of the preceding claims, for use in the treatment of cancer according to claim 1, wherein the cancer is selected from the group consisting of cancer types arising in the skin or in tissues that line or cover internal organs, such as skin, lung, colon, pancreatic, ovarian, epithelial, squamous and basal cell carcinomas, melanomas, papillomas, and adenomas.

5. A pharmaceutical composition comprising a hydroxyethyl starch according to any of the claims 1 to 3, for use in the treatment of cancer according to claim 1, wherein the cancer is selected from the group consisting of breast carcinoma, lung carcinoma, prostate carcinoma and renal carcinoma.

6. A pharmaceutical composition comprising a hydroxyethyl starch according to claims 1 or 2, for use in the treatment of cancer according to claim 1, wherein the cancer is selected from the group consisting of bone cancer, soft tissue cancer, osteosarcoma, synovialsarcoma, chondrosarcoma, liposarcoma, angiosarcoma, rhabdhosarcoma and fibrosarcoma.

7. A pharmaceutical composition comprising a hydroxyethyl starch according to claims 1 or 2, for use in the treatment of cancer according to claim 1, wherein the cancer is selected from the group consisting of biliary cancer, bladder cancer, breast cancer, cervical cancer, colorectal cancer, gastrointestinal cancer, malignant melanoma, mesothelioma, non-small cell lung cancer, pancreatic cancer, prostate cancer, sarcoma and small cell lung cancer.

8. A pharmaceutical composition comprising a hydroxyethyl starch according to any of the preceding claims, for use in the treatment of cancer according to any of claims 1 to 7, wherein the cancer is characterized as presenting a tumor type, **characterised by** a median doubling time of below 6 days, preferably of below or equal to 4 days.

9. A pharmaceutical composition comprising a hydroxyethyl starch according to claim 8, for use in the treatment of cancer according to claim 8, **characterized in that** the doubling time is determined in a mouse model suitable to determine cancer growth rates.

10. A pharmaceutical composition comprising a hydroxyethyl starch (HES) according to any of the preceding claims, for use in the treatment of cancer according to any of claims 1 to 9, wherein the hydroxyethyl starch has a mean molecular weight Mw between 20 and 1300 kDa determined according to the calibration method described in the European Pharmacopeia 7.0, 01/2011:1785, p. 984, with a dn/dc value of 0.147+/-0.001.

11. A pharmaceutical composition comprising a hydroxyethyl starch (HES) according to any of the preceding claims, for use in the treatment of cancer according to any of claims 1 to 9, wherein the hydroxyethyl starch has a mean

molecular weight Mw between 65 and 1300 kDa.

**12.** A pharmaceutical composition comprising a hydroxyethyl starch (HES) according to any of the preceding claims for use in the treatment of cancer according to any of claims 1 to 9, wherein the treatment comprises inhibiting or decelerating the proliferation of a tumor cell, whilst not affecting a normally growing cell.

**13.** A pharmaceutical composition comprising a hydroxyethyl starch (HES) according to any of the preceding claims for use in the treatment of cancer according to any of claims 1 to 9, wherein the treatment comprises arresting the mitotic cycle of the tumor cell.

**Patentansprüche**

**1.** Pharmazeutische Zusammensetzung, umfassend eine Hydroxyethylstärke (HES), wobei HES die einzige therapeutisch wirksame Verbindung zur Verwendung bei der Behandlung von Krebs ist, wobei die Behandlung als Reduktion von Tumorwachstumsraten gekennzeichnet ist.

**2.** Pharmazeutische Zusammensetzung, umfassend eine Hydroxyethylstärke (HES), nach Anspruch 1 zur Verwendung bei der Behandlung von Krebs nach Anspruch 1, wobei der Krebs aus der Gruppe der soliden Tumoren ausgewählt ist, bei denen es sich um Tumoren handelt, die in festen Geweben entstehen.

**3.** Pharmazeutische Zusammensetzung, umfassend eine Hydroxyethylstärke (HES), nach Anspruch 1 oder 2 zur Verwendung bei der Behandlung von Krebs nach Anspruch 1, wobei der Krebs aus der Gruppe der Karzinome ausgewählt ist.

**4.** Pharmazeutische Zusammensetzung, umfassend eine Hydroxyethylstärke, nach einem der vorhergehenden Ansprüche zur Verwendung bei der Behandlung von Krebs nach Anspruch 1, wobei der Krebs aus der Gruppe bestehend aus Krebsarten, die in der Haut oder in Geweben, die innere Organe säumen oder bedecken, entstehen, wie etwa Haut-, Lungen-, Kolon-, Pankreas-, Ovarial, Epithel-, Plattenepithel- und Basalzellkarzinome, Melanome, Papillome und Adenome, ausgewählt ist.

**5.** Pharmazeutische Zusammensetzung, umfassend eine Hydroxyethylstärke, nach einem der Ansprüche 1 bis 3 zur Verwendung bei der Behandlung von Krebs nach Anspruch 1, wobei der Krebs aus der Gruppe bestehend aus Brustkarzinom, Lungenkarzinom, Prostatakarzinom und Nierenkarzinom ausgewählt ist.

**6.** Pharmazeutische Zusammensetzung, umfassend eine Hydroxyethylstärke, nach Anspruch 1 oder 2 zur Verwendung bei der Behandlung von Krebs nach Anspruch 1, wobei der Krebs aus der Gruppe bestehend aus Knochenkrebs, Weichteilkrebs, Osteosarkom, Synovialsarkom, Chondrosarkom, Liposarkom, Angiosarkom, Rhabdhosarkom und Fibrosarkom ausgewählt ist.

**7.** Pharmazeutische Zusammensetzung, umfassend eine Hydroxyethylstärke, nach Anspruch 1 oder 2 zur Verwendung bei der Behandlung von Krebs nach Anspruch 1, wobei der Krebs aus der Gruppe bestehend aus Krebs des Gallenwegsystems, Blasenkrebs, Brustkrebs, Gebärmutterhalskrebs, Kolorektalkarzinom, Krebs des Magen-Darm-Trakts, malignem Melanom, Mesotheliom, nichtkleinzelligem Lungenkrebs, Bauchspeicheldrüsenkrebs, Prostatakrebs, Sarkom und kleinzelligem Lungenkrebs ausgewählt ist.

**8.** Pharmazeutische Zusammensetzung, umfassend eine Hydroxyethylstärke, nach einem der vorhergehenden Ansprüche zur Verwendung bei der Behandlung von Krebs nach einem der Ansprüche 1 bis 7, wobei der Krebs als einen Tumortyp präsentierend gekennzeichnet ist, der durch eine mediane Verdopplungszeit von unter 6 Tagen, vorzugsweise von unter oder gleich 4 Tagen gekennzeichnet ist.

**9.** Pharmazeutische Zusammensetzung, umfassend eine Hydroxyethylstärke, nach Anspruch 8 zur Verwendung bei der Behandlung von Krebs nach Anspruch 8, **dadurch gekennzeichnet, dass** die Verdopplungszeit in einem zur Bestimmung von Krebswachstumsgeschwindigkeiten geeigneten Mausmodell bestimmt wird.

**10.** Pharmazeutische Zusammensetzung, umfassend eine Hydroxyethylstärke (HES), nach einem der vorhergehenden Ansprüche zur Verwendung bei der Behandlung von Krebs nach einem der Ansprüche 1 bis 9, wobei die Hydroxyethylstärke ein mittleres Molekulargewicht Mw zwischen 20 und 1300 kDa aufweist, bestimmt gemäß dem in der

europäischen Pharmakopöe 7.0, 01/2011:1785, S. 984, beschriebenen Kalibrierungsverfahren bei einem dn/dc-Wert von 0,147+/-0,001.

**11.** Pharmazeutische Zusammensetzung, umfassend eine Hydroxyethylstärke (HES), nach einem der vorhergehenden Ansprüche zur Verwendung bei der Behandlung von Krebs nach einem der Ansprüche 1 bis 9, wobei die Hydroxyethylstärke ein mittleres Molekulargewicht Mw zwischen 65 und 1300 kDa aufweist.

**12.** Pharmazeutische Zusammensetzung, umfassend eine Hydroxyethylstärke (HES), nach einem der vorhergehenden Ansprüche zur Verwendung bei der Behandlung von Krebs nach einem der Ansprüche 1 bis 9, wobei die Behandlung Hemmen oder Verlangsamen der Proliferation einer Tumorzelle ohne Auswirkung auf eine normal wachsende Zelle umfasst.

**13.** Pharmazeutische Zusammensetzung, umfassend eine Hydroxyethylstärke (HES), nach einem der vorhergehenden Ansprüche zur Verwendung bei der Behandlung von Krebs nach einem der Ansprüche 1 bis 9, wobei die Behandlung Anhalten des Mitosezyklus der Tumorzelle umfasst.

## Revendications

**1.** Composition pharmaceutique comprenant un hydroxyéthyl-amidon (HES), dans laquelle HES est le seul composé thérapeutiquement actif pour utilisation dans le traitement d'un cancer, le traitement étant **caractérisé par** une réduction des taux de croissance tumoraux.

**2.** Composition pharmaceutique comprenant un hydroxyéthyl-amidon (HES) selon la revendication 1, pour utilisation dans le traitement d'un cancer selon la revendication 1, le cancer étant choisi dans le groupe des tumeurs solides, qui sont des tumeurs survenant dans des tissus solides.

**3.** Composition pharmaceutique comprenant un hydroxyéthyl-amidon (HES) selon les revendications 1 ou 2, pour utilisation dans le traitement d'un cancer selon la revendication 1, le cancer étant choisi dans le groupe des carcinomes.

**4.** Composition pharmaceutique comprenant un hydroxyéthyl-amidon selon l'une quelconque des revendications précédentes, pour utilisation dans le traitement d'un cancer selon la revendication 1, le cancer étant choisi dans le groupe constitué de types de cancer survenant dans la peau ou dans des tissus qui revêtent ou recouvrent des organes internes, tels que des cancers de la peau, du poumon, du côlon, du pancréas, de l'ovaire, de l'épithélium, des carcinomes épidermoïdes et à cellules basales, des mélanomes, des papillomes et des adénomes.

**5.** Composition pharmaceutique comprenant un hydroxyéthyl-amidon selon l'une quelconque des revendications 1 à 3, pour utilisation dans le traitement d'un cancer selon la revendication 1, le cancer étant choisi dans le groupe constitué d'un carcinome du sein, un carcinome du poumon, un carcinome de la prostate et un carcinome rénal.

**6.** Composition pharmaceutique comprenant un hydroxyéthyl-amidon selon les revendications 1 ou 2, pour utilisation dans le traitement d'un cancer selon la revendication 1, le cancer étant choisi dans le groupe constitué d'un cancer des os, un cancer des tissus mous, un ostéosarcome, un sarcome synovial, un chondrosarcome, un liposarcome, un angiosarcome, un rhabdomyosarcome et un fibrosarcome.

**7.** Composition pharmaceutique comprenant un hydroxyéthyl-amidon selon les revendications 1 ou 2, pour utilisation dans le traitement d'un cancer selon la revendication 1, le cancer étant choisi dans le groupe constitué d'un cancer des voies biliaires, un cancer de la vessie, un cancer du sein, un cancer du col de l'utérus, un cancer colorectal, un cancer gastro-intestinal, un mélanome malin, un mésothéliome, un cancer du poumon non à petites cellules, un cancer du pancréas, un cancer de la prostate, un sarcome et un cancer du poumon à petites cellules.

**8.** Composition pharmaceutique comprenant un hydroxyéthyl-amidon selon l'une quelconque des revendications précédentes, pour utilisation dans le traitement d'un cancer selon l'une quelconque des revendications 1 à 7, le cancer étant **caractérisé en ce qu'**il présente un type de tumeur, **caractérisé par** un temps de doublement médian inférieur à 6 jours, de préférence inférieur ou égal à 4 jours.

**9.** Composition pharmaceutique comprenant un hydroxyéthyl-amidon selon la revendication 8, pour utilisation dans

le traitement d'un cancer selon la revendication 8, **caractérisée en ce que** le temps de doublement est déterminé dans un modèle de souris adapté pour déterminer les taux de croissance d'un cancer.

**10.** Composition pharmaceutique comprenant un hydroxyéthyl-amidon (HES) selon l'une quelconque des revendications précédentes, pour utilisation dans le traitement d'un cancer selon l'une quelconque des revendications 1 à 9, dans laquelle l'hydroxyéthyl-amidon a un poids moléculaire moyen Mw compris entre 20 et 1300 kDa déterminé selon le procédé d'étalonnage décrit dans la pharmacopée européenne 7.0, 01/2011:1785, p. 984, avec une valeur dn/dc de 0,147+/-0,001.

**11.** Composition pharmaceutique comprenant un hydroxyéthyl-amidon (HES) selon l'une quelconque des revendications précédentes, pour utilisation dans le traitement d'un cancer selon l'une quelconque des revendications 1 à 9, dans laquelle l'hydroxyéthyl-amidon a un poids moléculaire moyen Mw compris entre 65 et 1300 kDa.

**12.** Composition pharmaceutique comprenant un hydroxyéthyl-amidon (HES) selon l'une quelconque des revendications précédentes pour utilisation dans le traitement d'un cancer selon l'une quelconque des revendications 1 à 9, le traitement comprenant l'inhibition ou la décélération de la prolifération d'une cellule tumorale, tout en n'ayant aucun effet sur une cellule croissant normalement.

**13.** Composition pharmaceutique comprenant un hydroxyéthyl-amidon (HES) selon l'une quelconque des revendications précédentes pour utilisation dans le traitement d'un cancer selon l'une quelconque des revendications 1 à 9, le traitement comprenant l'arrêt du cycle mitotique de la cellule tumorale.

**Figure 1:**

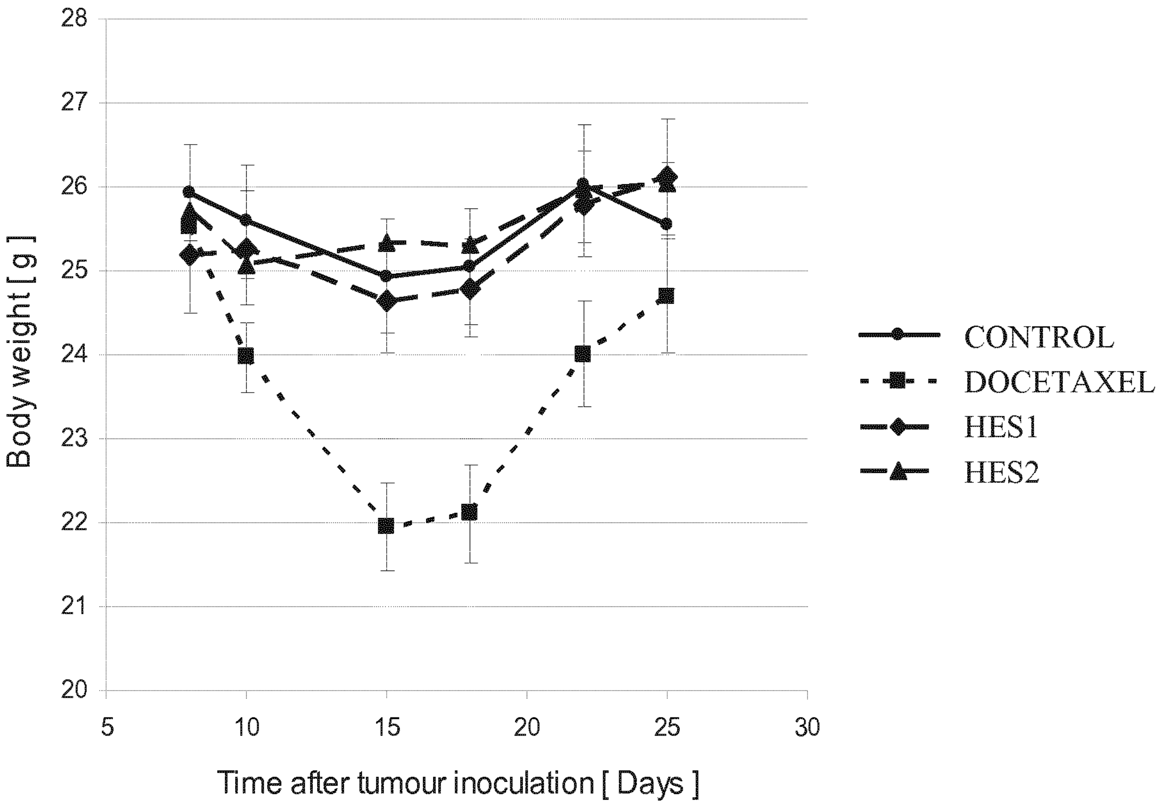
28
27
26
25
24
23
22
21
20
Body weight [ g ]
5
10
15
20
25
30
Time after tumour inoculation [ Days ]
CONTROL
DOCETAXEL
HES1
HES2

Figure. 2:

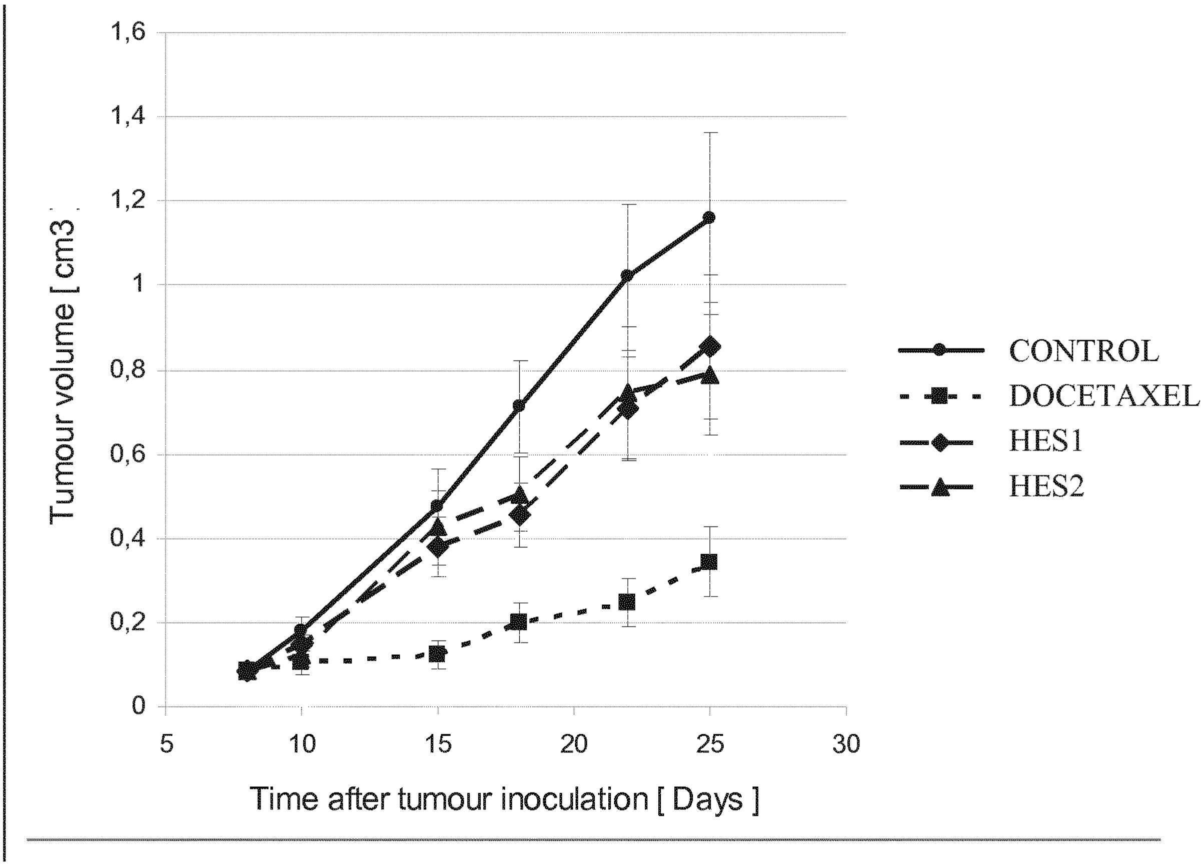
1,6
1,4
1,2
1
0,8
0,6
0,4
0,2
0
Tumour volume [ cm3 ]
5
10
15
20
25
30
Time after tumour inoculation [ Days ]
CONTROL
DOCETAXEL
HES1
HES2

**Figure 3:**

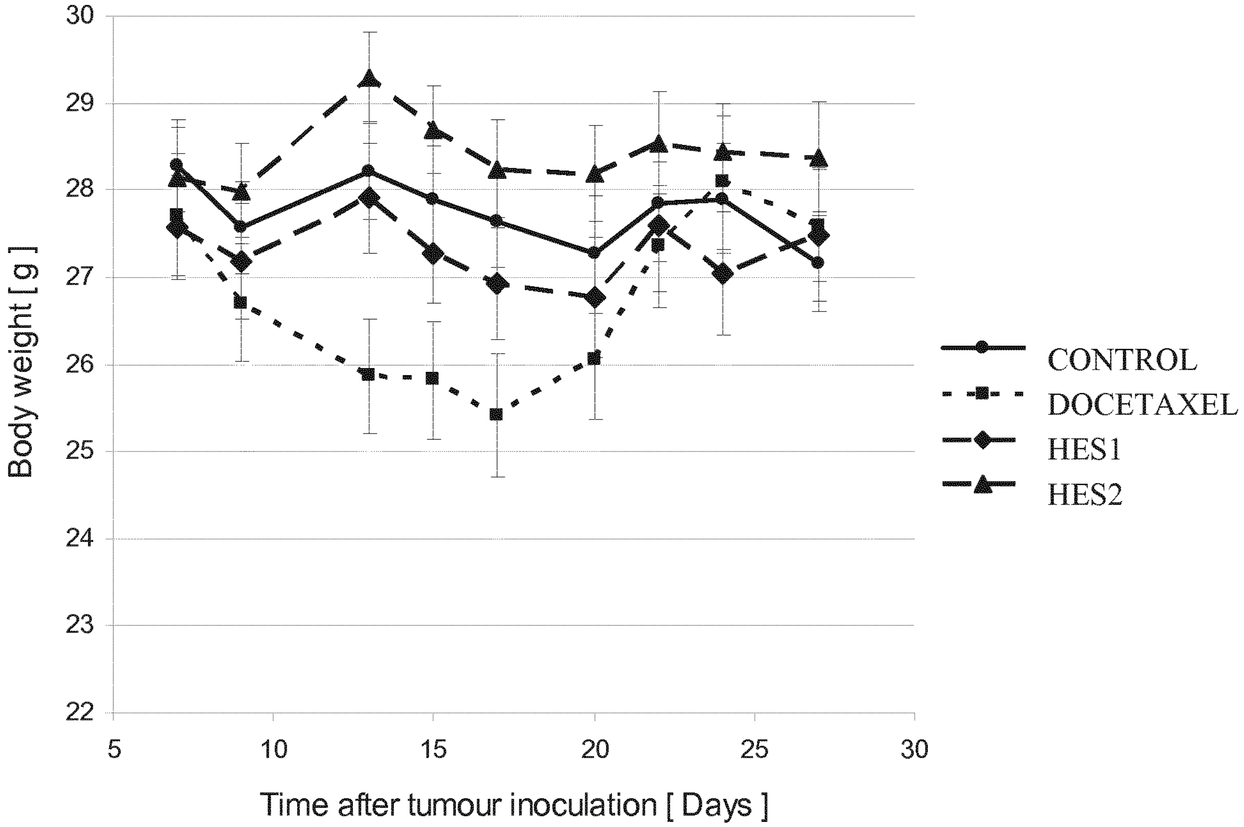
30
29
28
27
26
25
24
23
22
Body weight [ g ]
5
10
15
20
25
30
Time after tumour inoculation [ Days ]
CONTROL
DOCETAXEL
HES1
HES2

**Figure 4:**

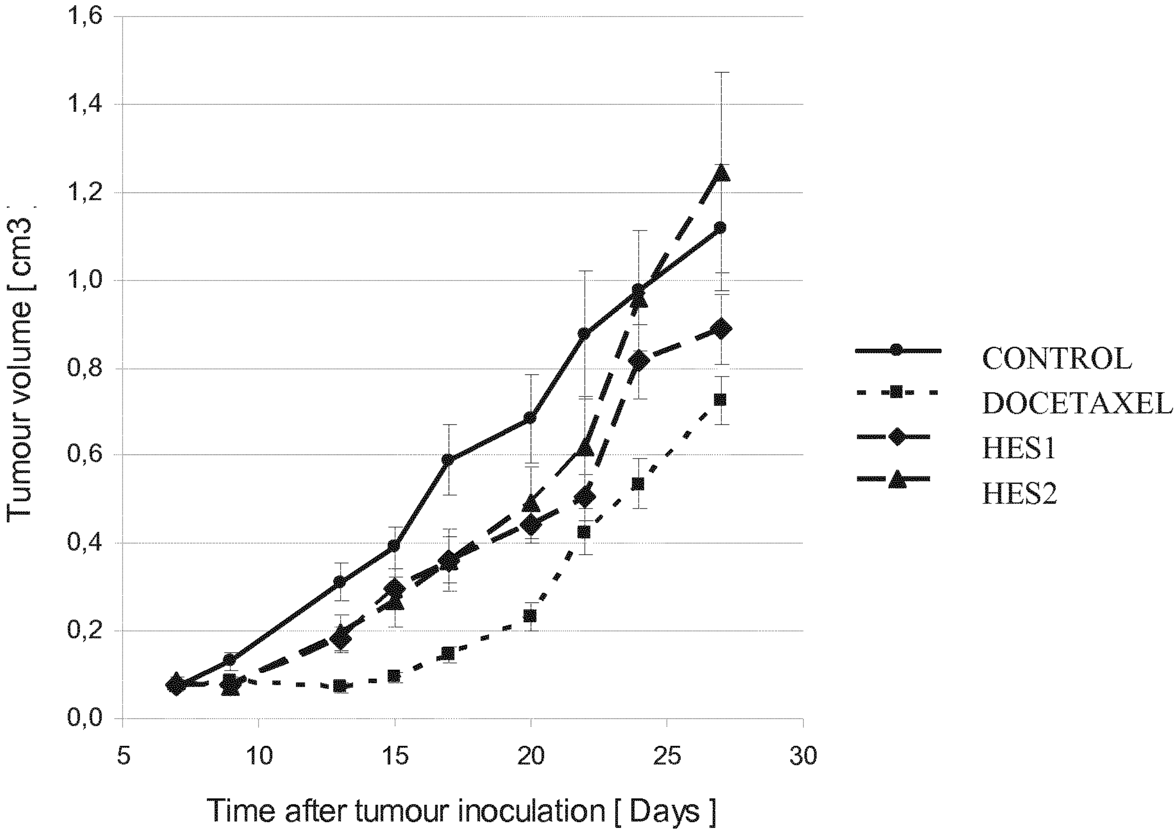
1,6
1,4
1,2
1,0
0,8
0,6
0,4
0,2
0,0
Tumour volume [ cm3 ]
5
10
15
20
25
30
Time after tumour inoculation [ Days ]
CONTROL
DOCETAXEL
HES1
HES2

Figure 5

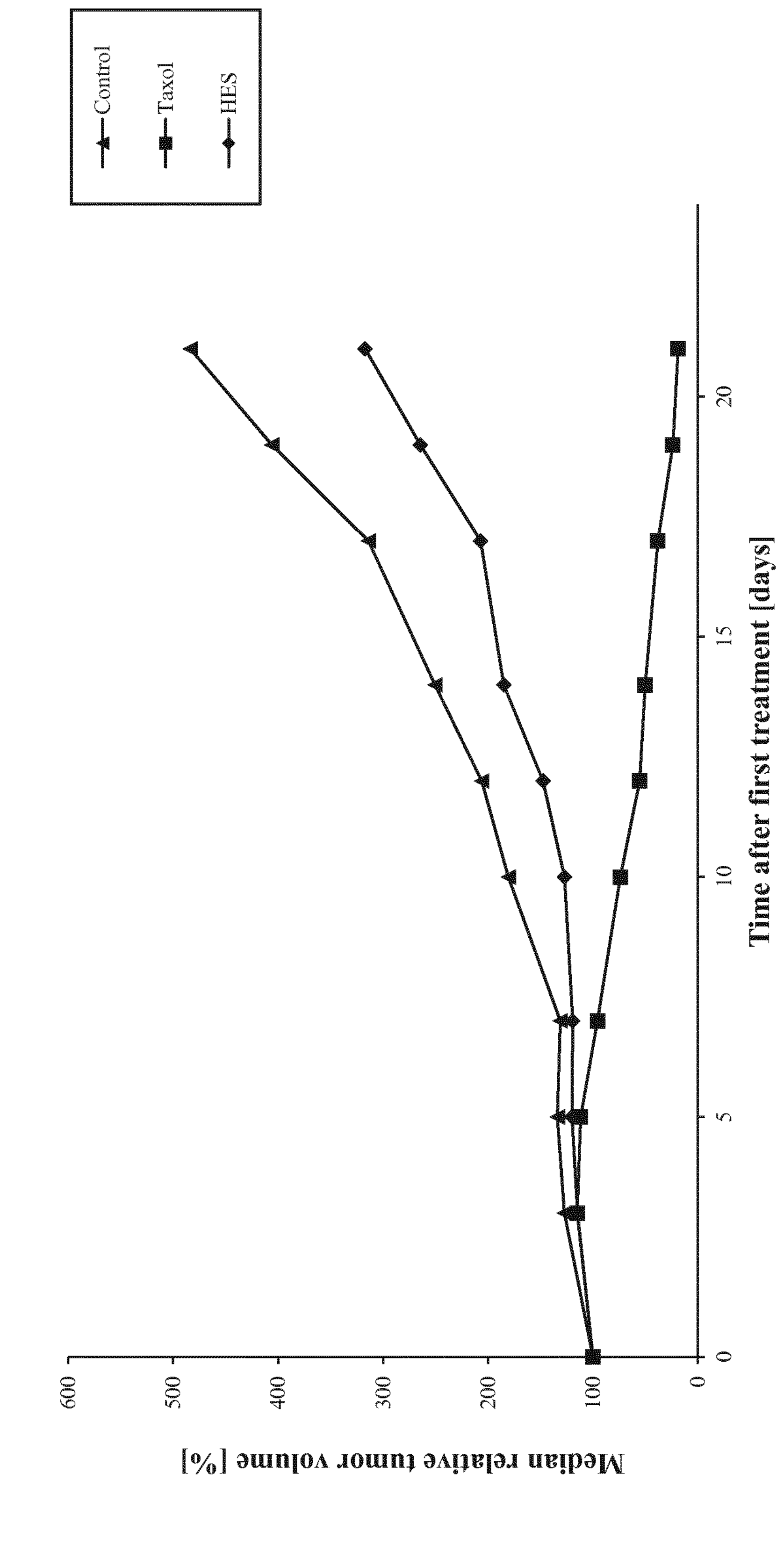
Control
Taxol
HES
Median relative tumor volume [%]
0
100
200
300
400
500
600
Time after first treatment [days]
0
5
10
15
20

Figure 6

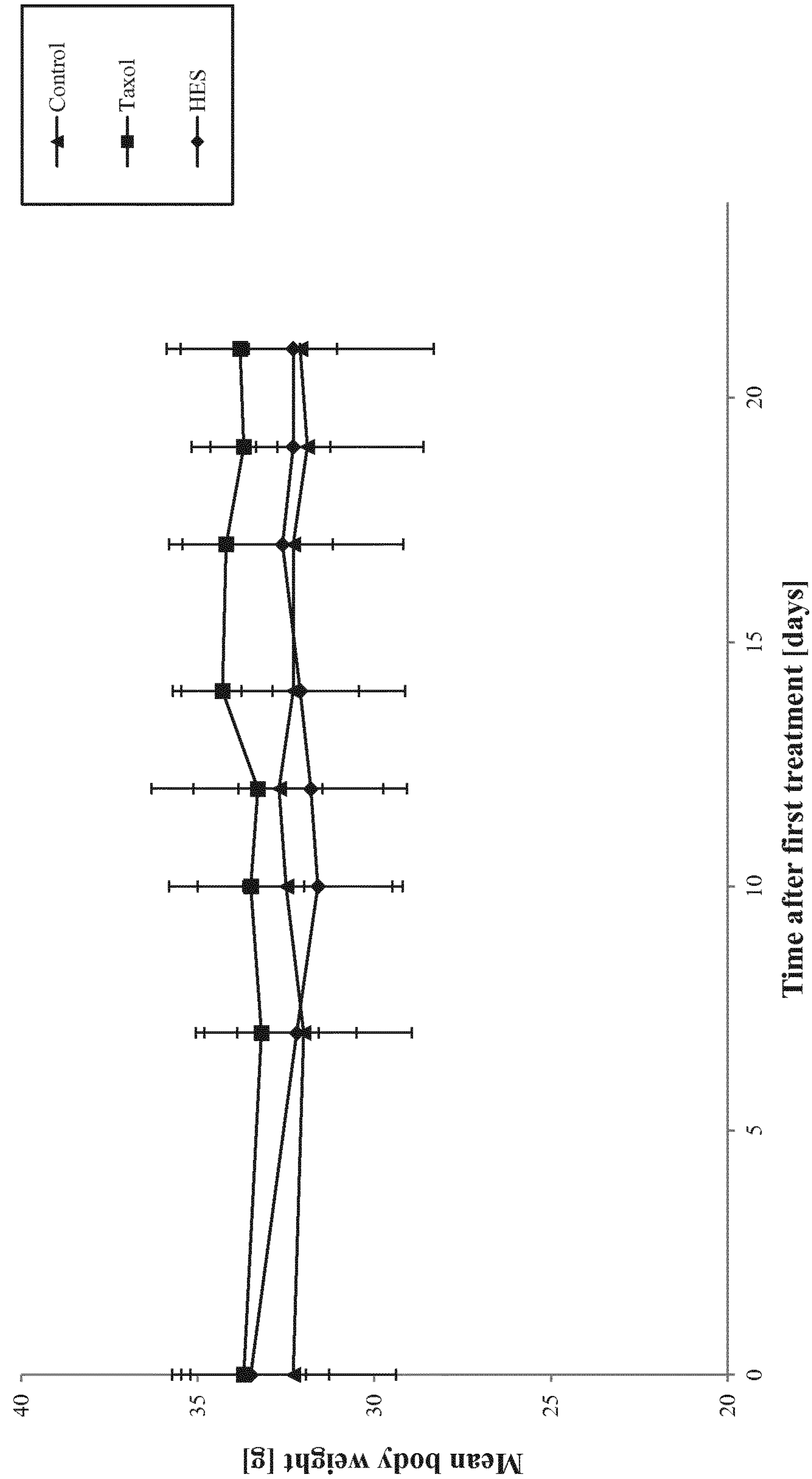
Control
Taxol
HES
Mean body weight [g]
40
35
30
25
20
0
5
10
15
20
Time after first treatment [days]

Figure 7

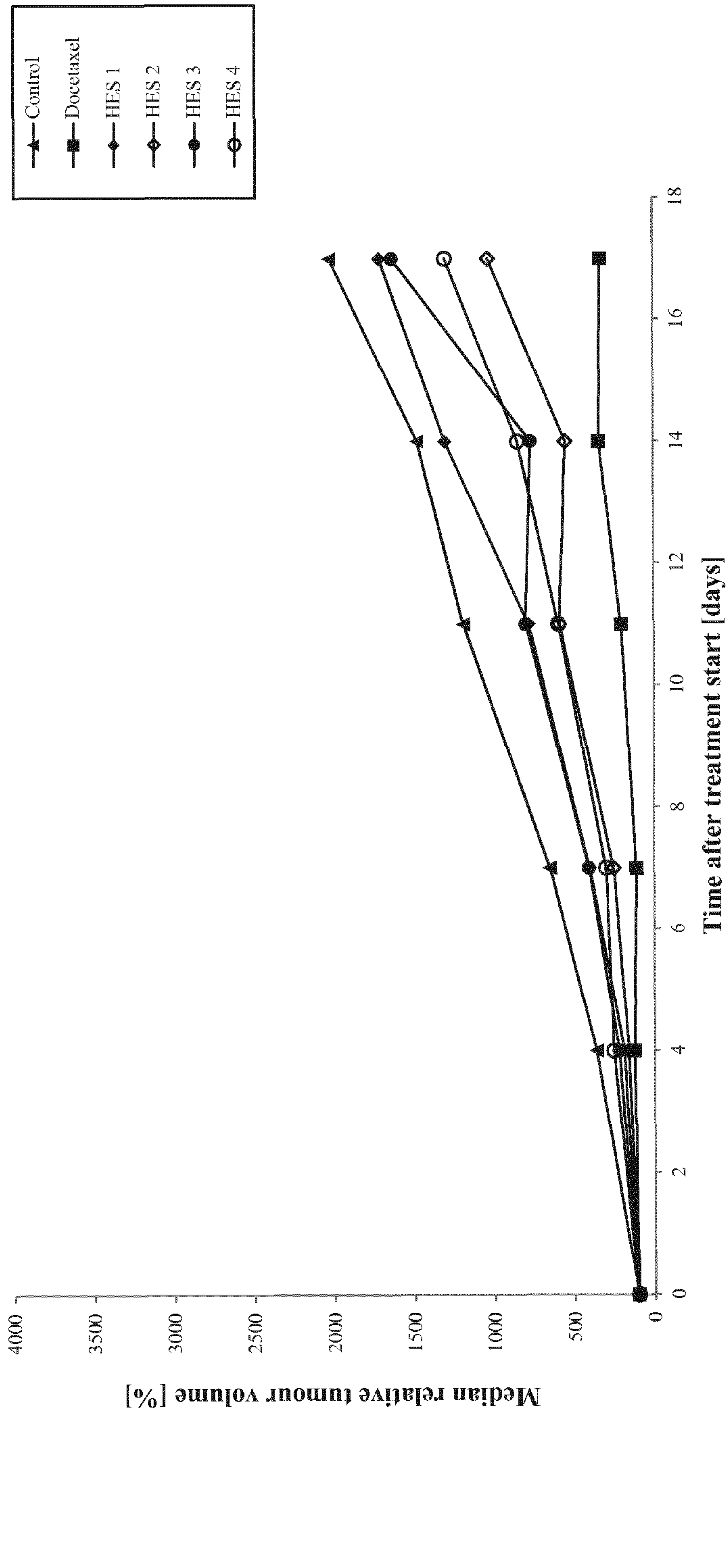
Control
Docetaxel
HES 1
HES 2
HES 3
HES 4
Median relative tumour volume [%]
Time after treatment start [days]
0
500
1000
1500
2000
2500
3000
3500
4000
0
2
4
6
8
10
12
14
16
18

Figure 8

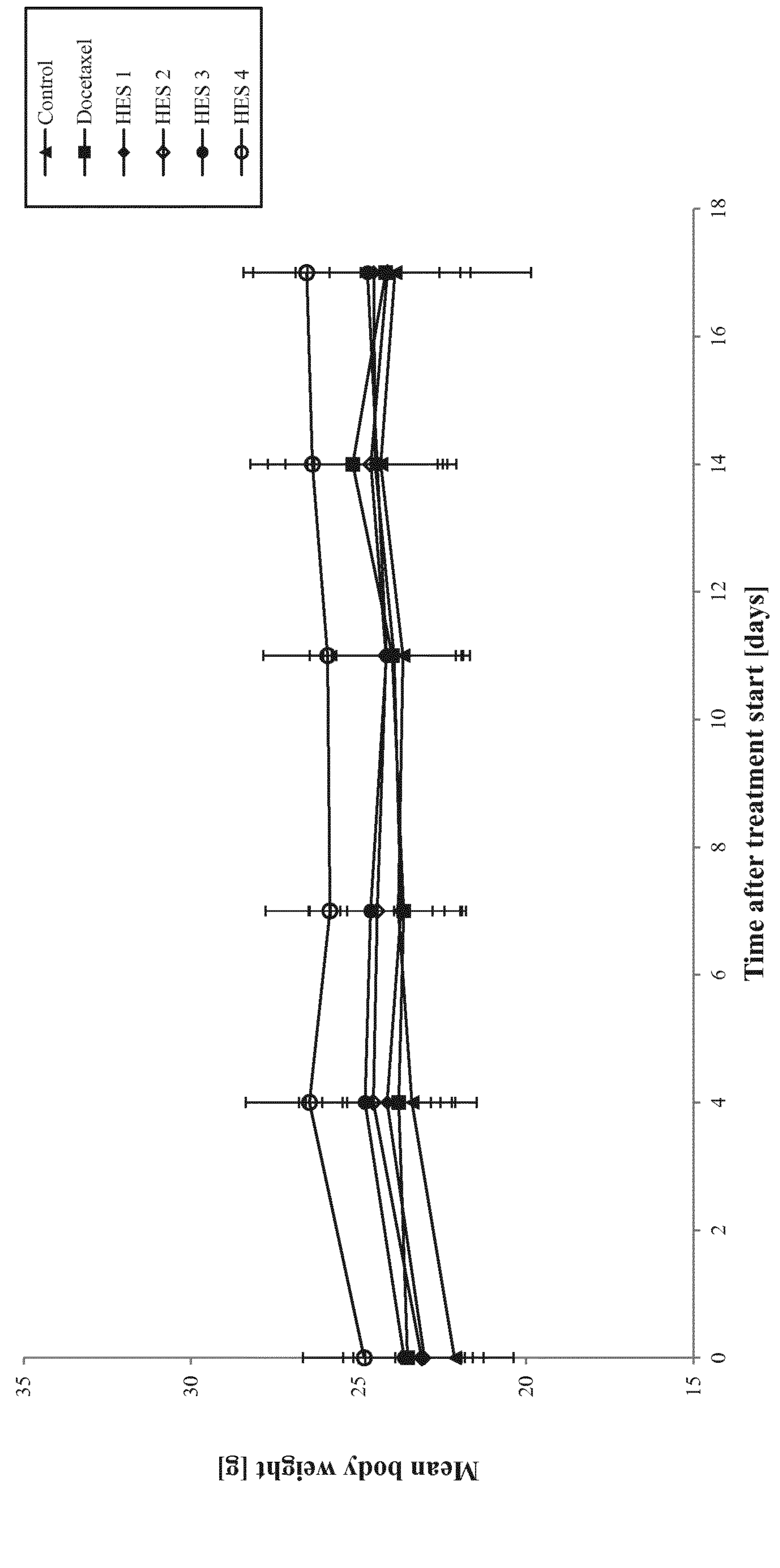
Mean body weight [g]
35
30
25
20
15
0
2
4
6
8
10
12
14
16
18
Time after treatment start [days]
Control
Docetaxel
HES 1
HES 2
HES 3
HES 4

Figure 9

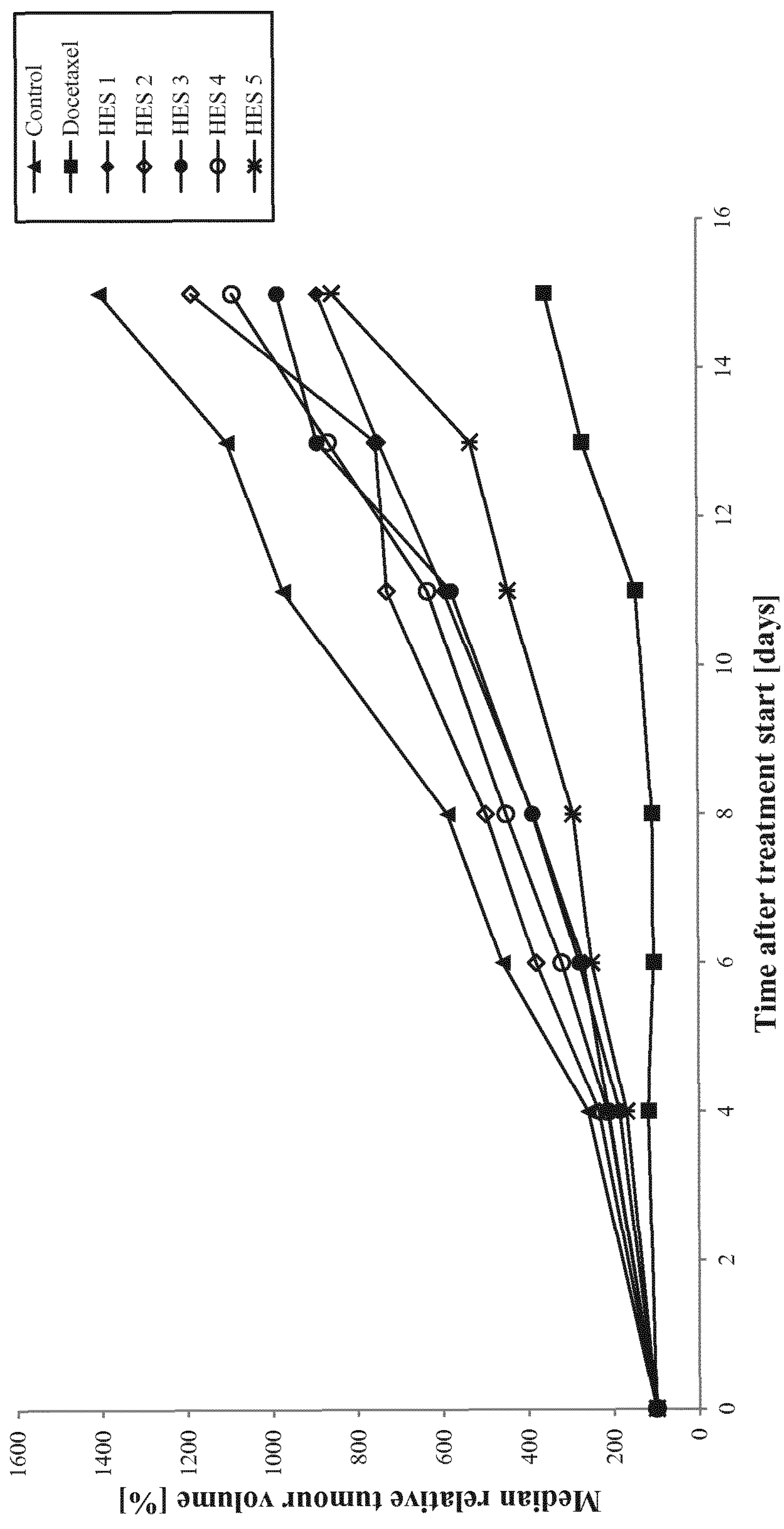
Median relative tumour volume [%]
0
200
400
600
800
1000
1200
1400
1600
Time after treatment start [days]
0
2
4
6
8
10
12
14
16
Control
Docetaxel
HES 1
HES 2
HES 3
HES 4
HES 5

Figure 10

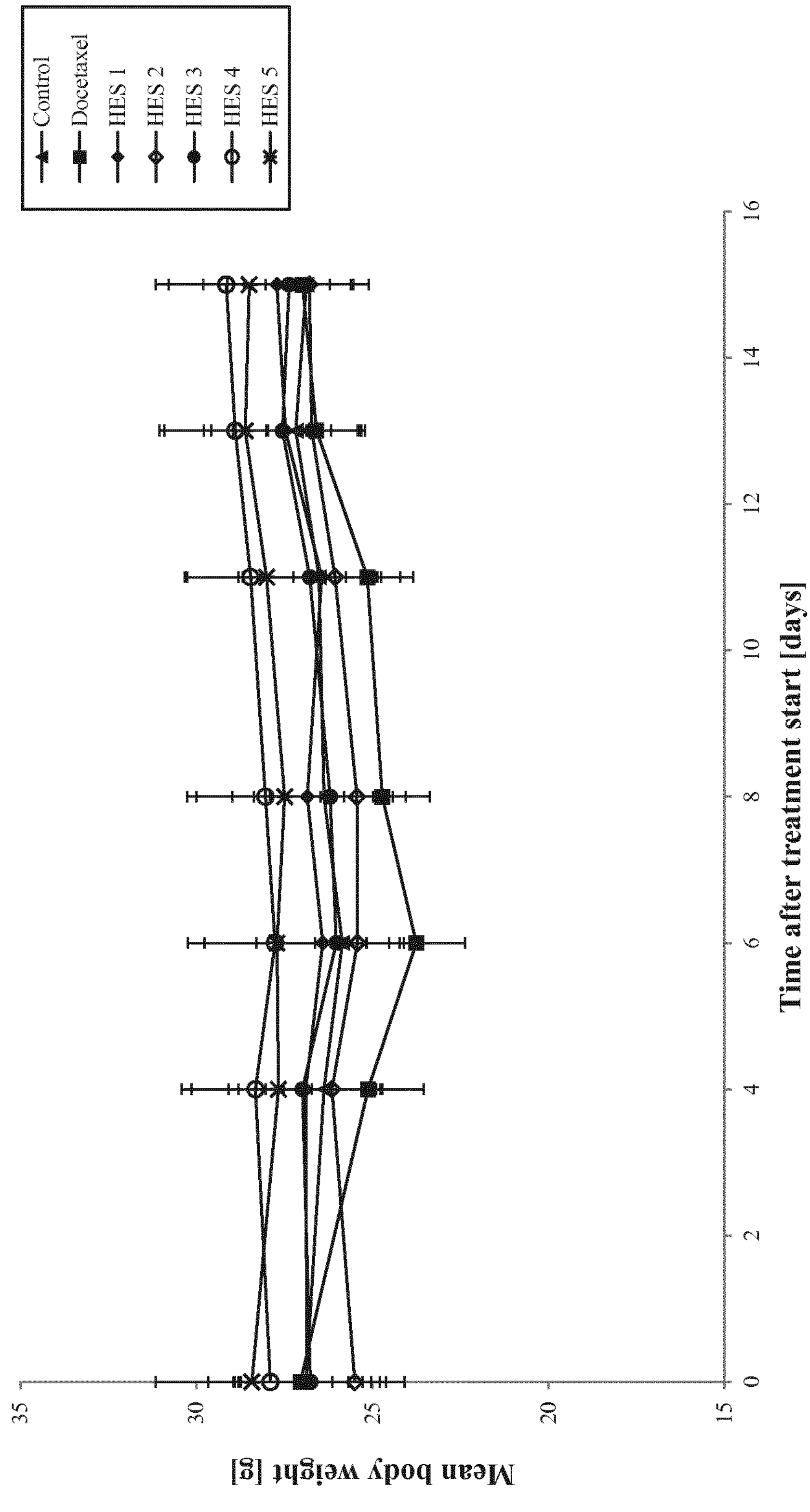
Mean body weight [g]
35
30
25
20
15
0
2
4
6
8
10
12
14
16
Time after treatment start [days]
Control
Docetaxel
HES 1
HES 2
HES 3
HES 4
HES 5

Figure 11

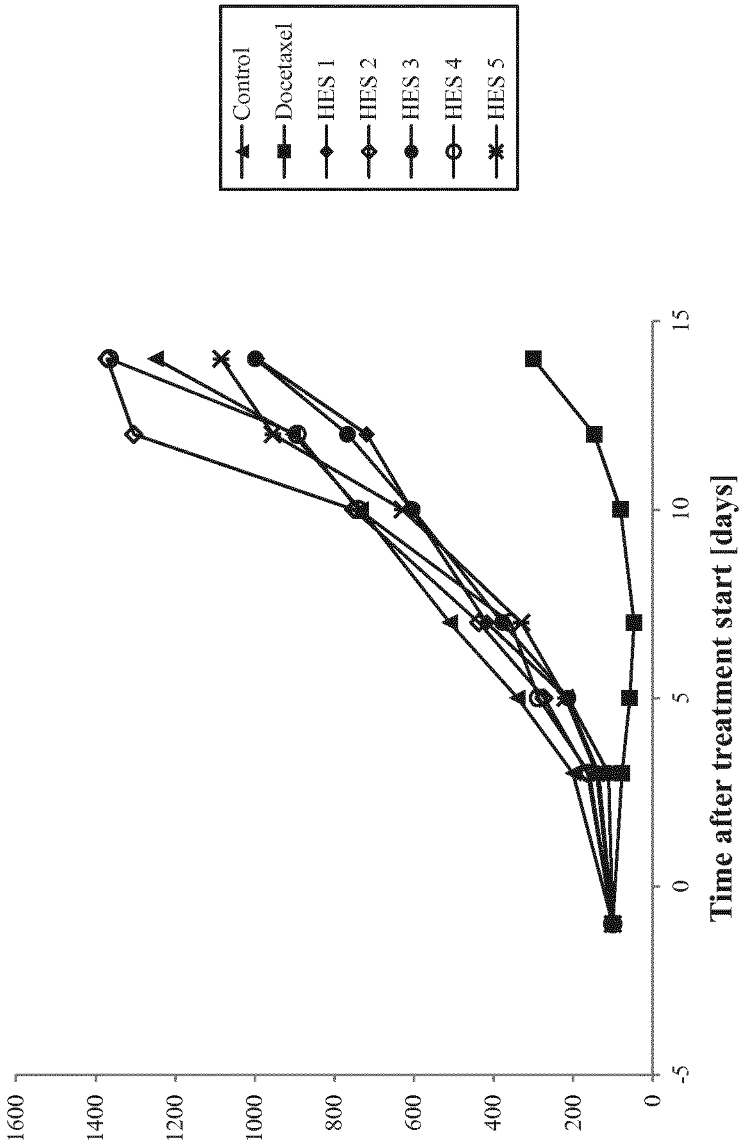
Relative median tumour volume (%)
1600
1400
1200
1000
800
600
400
200
0
-5
0
5
10
15
Time after treatment start [days]
Control
Docetaxel
HES 1
HES 2
HES 3
HES 4
HES 5

Figure 12

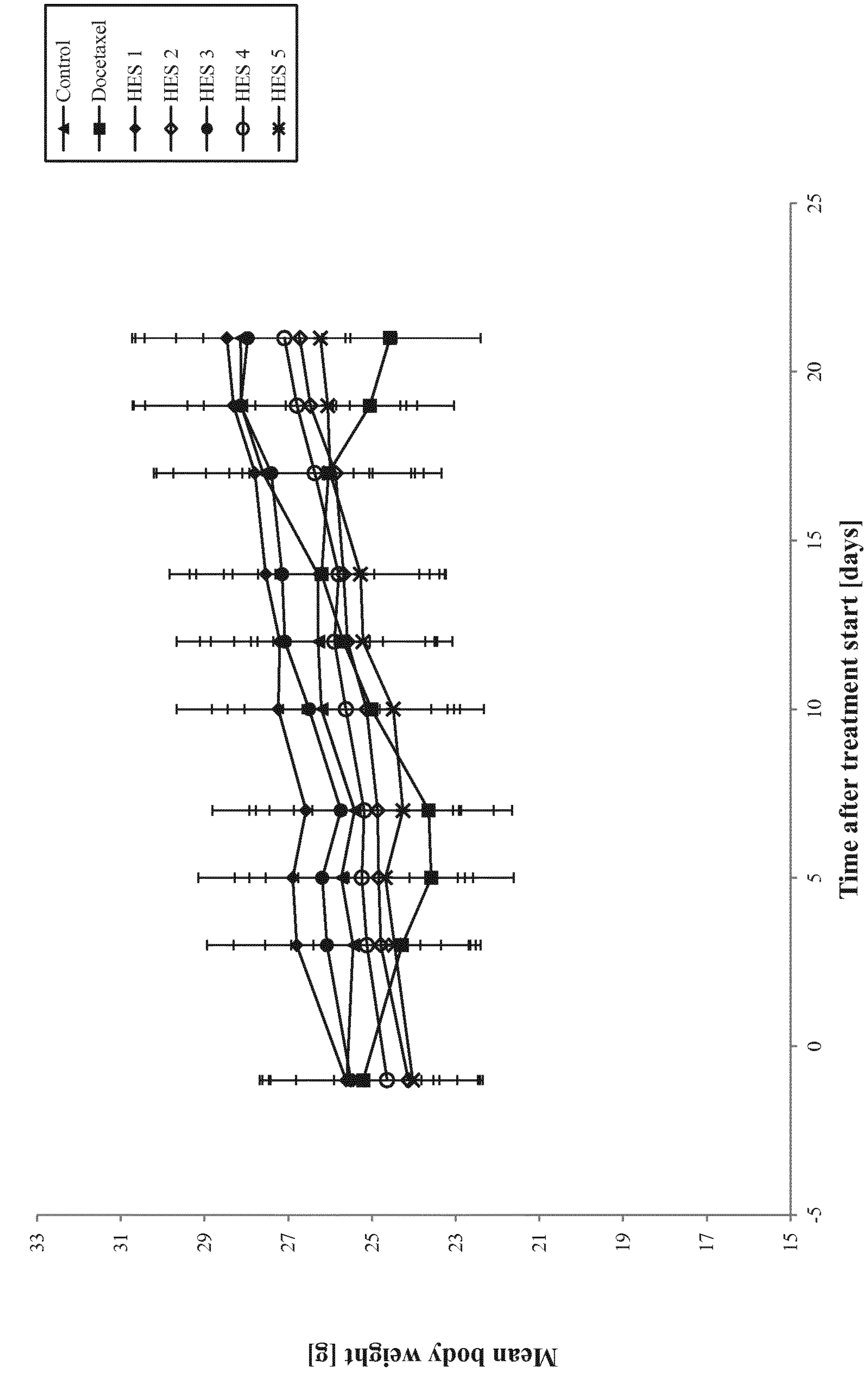
Mean body weight [g]
33
31
29
27
25
23
21
19
17
15
-5
0
5
10
15
20
25
Time after treatment start [days]
Control
Docetaxel
HES 1
HES 2
HES 3
HES 4
HES 5

Figure 13

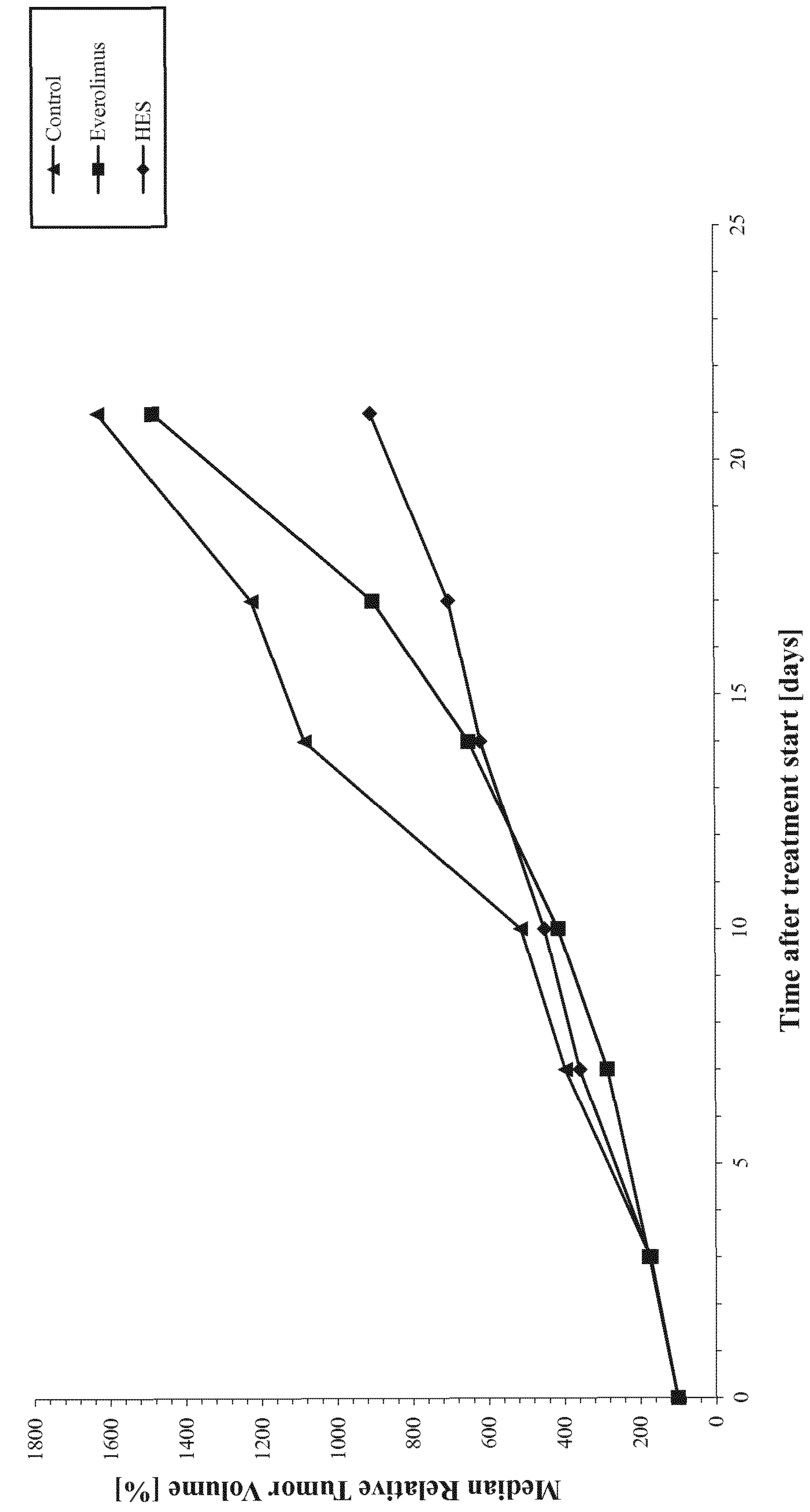
Control
Everolimus
HES
Median Relative Tumor Volume [%]
0
200
400
600
800
1000
1200
1400
1600
1800
Time after treatment start [days]
0
5
10
15
20
25

Figure 14

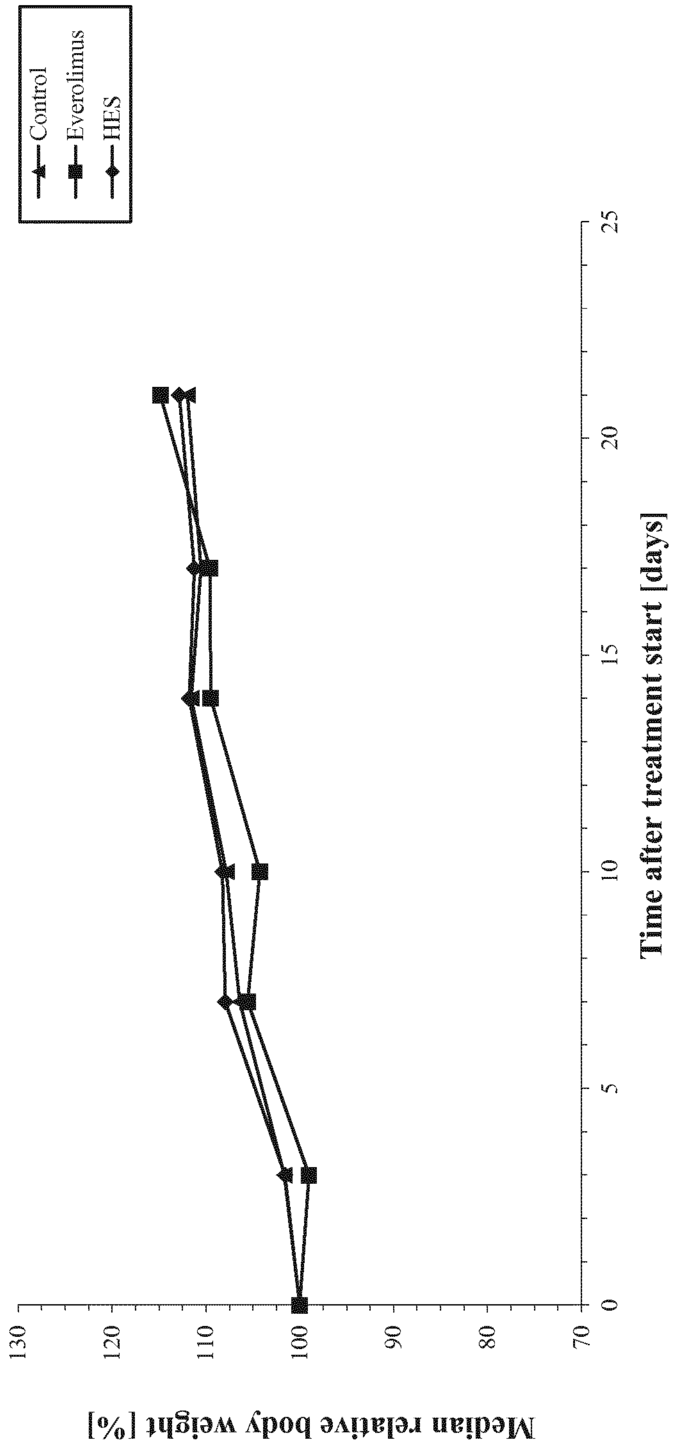
Median relative body weight [%]
130
120
110
100
90
80
70
0
5
10
15
20
25
Time after treatment start [days]
Control
Everolimus
HES

Figure 15

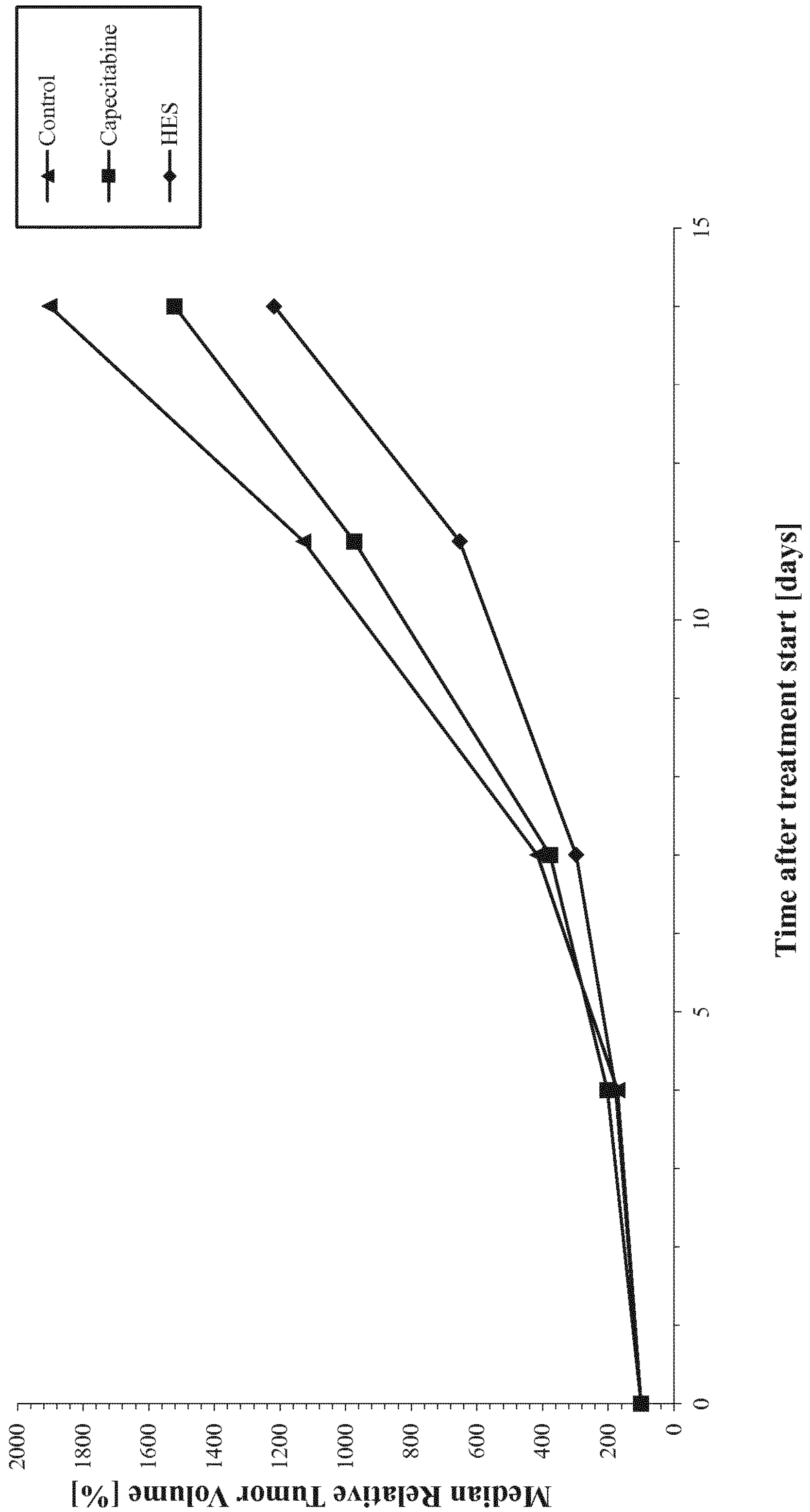
Control
Capecitabine
HES
Median Relative Tumor Volume [%]
0
200
400
600
800
1000
1200
1400
1600
1800
2000
Time after treatment start [days]
0
5
10
15

Figure 16

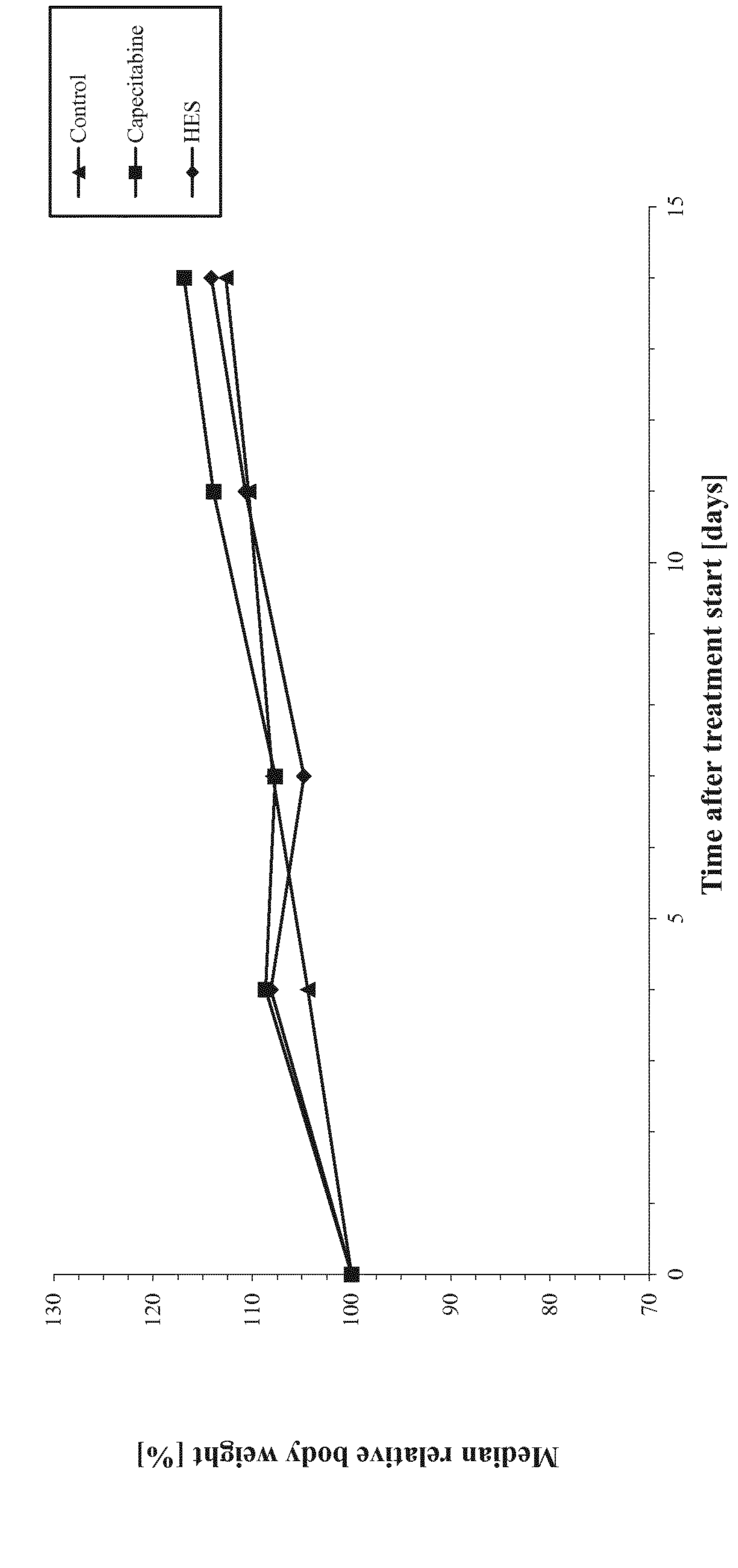

Control
Capecitabine
HES
Median relative body weight [%]
130
120
110
100
90
80
70
0
5
10
15
Time after treatment start [days]

Figure 17

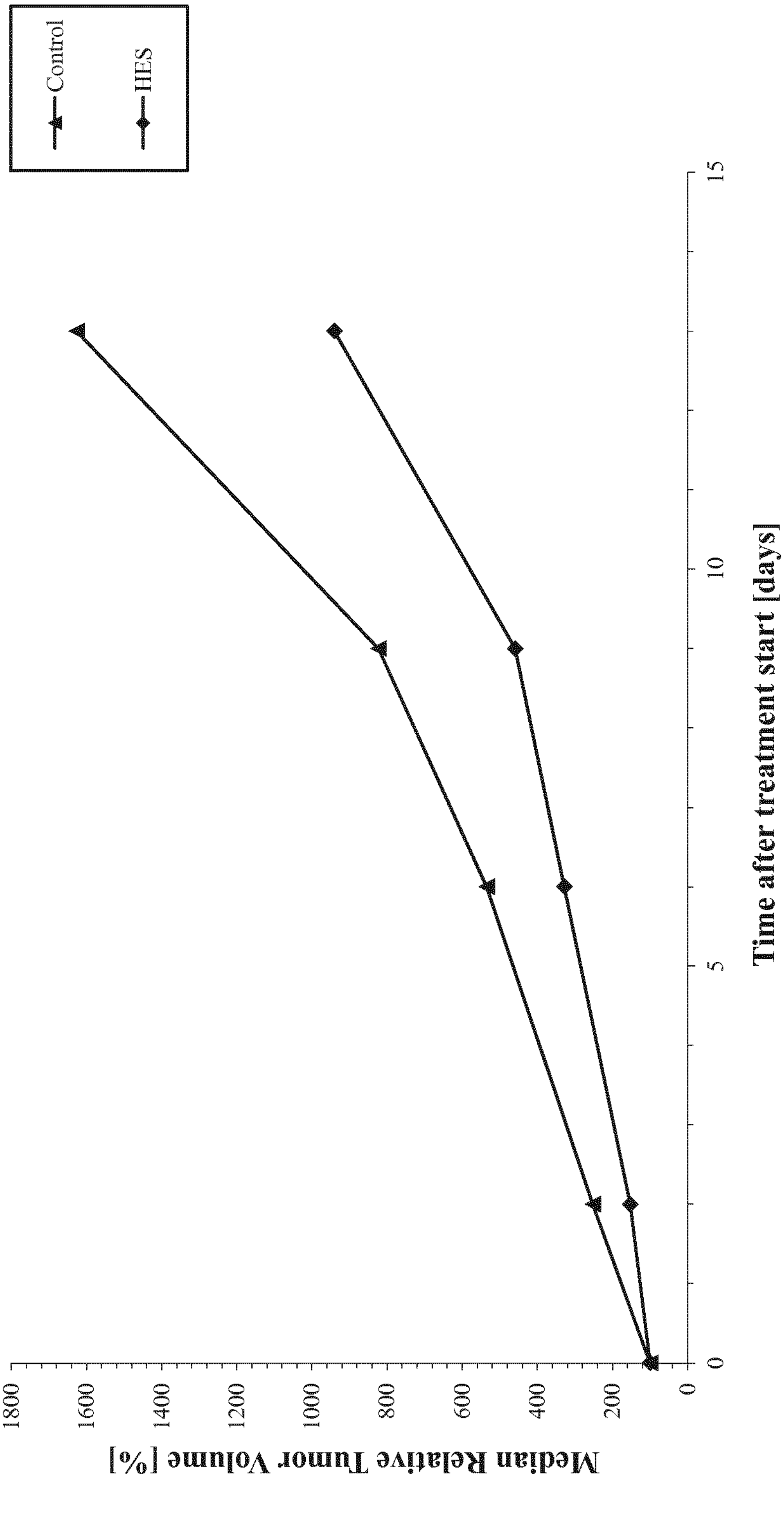

Median Relative Tumor Volume [%]
0
200
400
600
800
1000
1200
1400
1600
1800
Time after treatment start [days]
0
5
10
15
Control
HES

Figure 18

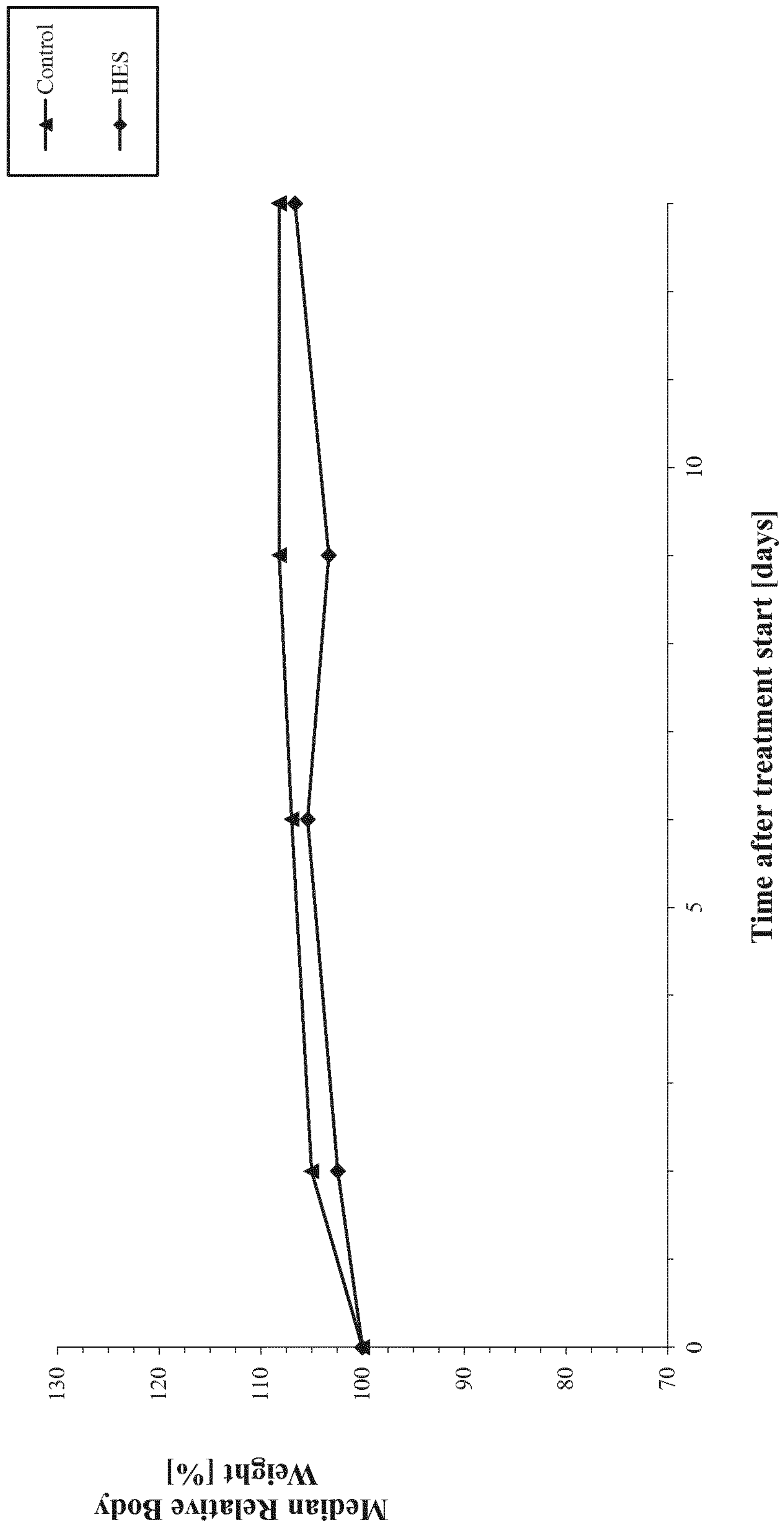

Control
HES
Median Relative Body Weight [%]
130
120
110
100
90
80
70
0
5
10
Time after treatment start [days]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 4023788, Schumann
- WO 9640108 A
- WO 03074088 A
- US 6207654 B, Zikria
- WO 0048637 A
- US 5502043 A
- EP 1732953 B
- EP 0402724B A
- US 6680305 B


### Non-patent literature cited in the description


- **SOMMERMEYER et al.** *Krankenhauspharmazie,* 1987, vol. 8 (8), 271-278
- **WEIDLER et al.** *Arzneimittelforschung/Drug Research,* 1991, vol. 41, 494-498
- **MOHAMED et al.** *European Journal of Surgical Oncology,* 2003, vol. 29, 261-265
- **SOMMERMEYER et al.** Chromatographia. 1988, vol. 25, 167-168
- **C. JUNGHEINRICH et al.** *Clin. Pharmacokin.,* 2005, vol. 44 (7), 681-699
- **J.-M. MISHLER IV.** Pharmacology of hydroxyethyl starches. Oxford Medical Publications, 2002, 1-30
- **W.-M. KULICKE et al.** *Starch,* 1993, vol. 45 (12), 445-450
- *European Pharmacopoeia 7.0,* January 2011, vol. 1785, 984
- **B. WITTGREN et al.** *Int. J. Polym. Anal. Charact.,* 2002, vol. 7 (1-2), 19-40
- **Y. M. LIU et al.** *Chin. Chem. Lett.,* 2002, vol. 13 (11), 1097-1099
- **W. BANKS et al.** *Br. J. Pharmac.,* 1973, vol. 47, 172-178
- **O. LARM et al.** *Starch,* 1981, vol. 33 (7), 240-244
- **SOMMERMEYER et al.** *Starch,* 1992, vol. 44 (6), 215-218
- **YING-CHE LEE et al.** *Anal. Chem.,* 1983, vol. 55, 334-338
- **K. L. HODGES et al.** *Anal. Chem.,* 1979, vol. 51, 2171
- **WAITZINGER et al.** *Clin. Drug Invest.,* 1998, vol. 16, 151-160
- **JUNGHEINRICH et al.** *Clin. Pharmacokinet.,* 2006, vol. 44 (7), 681-699
- **SOMMERMEYER et al.** *Krankenhauspharmazie,* 1987, vol. 8 (08), 271-278
- **A.N.BELDER ; B.NORMAN.** *Carbohydrate Research,* 1969, vol. 10, 391-394
- **NAUNDORF H. ; REWASOWA E. ; FICHTNER I et al.** Characterization of two human mammary carcinomas, MT-1 and MT-3, suitable for in vivo testing of ether lipids and their derivatives. *Breast Cancer Res Treat.,* 1992, vol. 23, 87-95
- **NAUNDORF H. ; REWASOWA E. ; FICHTNER I et al.** Characterization of two human mammary carcinomas, MT-1 and MT-3, suitable for in vivo testing of ether lipids and their derivatives. *Breast Cancer Res Treat,* 1992, vol. 23, 87-95